(19) Europäisches Patentamt / European Patent Office / Office européen des brevets

(11) **EP 2 751 556 B1**

(12) **EUROPEAN PATENT SPECIFICATION**

(45) Date of publication and mention of the grant of the patent:
**06.07.2016 Bulletin 2016/27**

(51) Int Cl.:
**G01N 27/74** (2006.01)   **G06F 19/10** (2011.01)

(21) Application number: **12836965.9**

(86) International application number:
**PCT/US2012/051870**

(22) Date of filing: **22.08.2012**

(87) International publication number:
**WO 2013/048644 (04.04.2013 Gazette 2013/14)**

(54) **DEVICE AND METHOD FOR DETECTION AND QUANTIFICATION OF IMMUNOLOGICAL PROTEINS, PATHOGENIC AND MICROBIAL AGENTS AND CELLS**

VORRICHTUNG UND VERFAHREN ZUM NACHWEIS UND ZUR QUANTIFIZIERUNG VON IMMUNOLOGISCHEN PROTEINEN SOWIE PATHOGENEN MIKROBIELLEN MITTELN UND ZELLEN

DISPOSITIF ET PROCÉDÉ DE DÉTECTION ET DE QUANTIFICATION DE PROTÉINES IMMUNOLOGIQUES, AGENTS ET CELLULES PATHOGÈNES ET MICROBIENS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.09.2011 US 201161539210 P**
**21.08.2012 US 201213590859**

(43) Date of publication of application:
**09.07.2014 Bulletin 2014/28**

(73) Proprietor: **Carnegie Mellon University**
**Pittsburgh, PA 15213-3890 (US)**

(72) Inventors:
• **GANDINI, Alberto**
  **Pittsburgh, Pennsylvania 15203 (US)**
• **ANTAKI, James, F.**
  **Allison Park, Pennsylvania 15101 (US)**

(74) Representative: **Gowshall, Jonathan Vallance**
**Forresters**
**Skygarden**
**Erika-Mann-Strasse 11**
**80636 München (DE)**

(56) References cited:
WO-A1-2005/010543    WO-A1-2009/093160
WO-A2-2008/002462    US-A1- 2009 181 411
US-A1- 2010 134 097    US-A1- 2011 212 440
US-A1- 2011 236 999    US-B2- 7 316 899
US-B2- 7 892 856

**Description**

FIELD OF THE INVENTION

The present invention relates to the field of immunoassay and microfluidic devices and, in particular, to a point-of-care diagnostic method and device for the detection and quantification of magnetic-responsive micro-beads conjugated with proteins, cells and microbial agents dispersed in a liquid sample.

BACKGROUND OF THE INVENTION

Current immunoassay technologies for the detection and quantification of proteins rely on the specificity of the chemical interaction between antigens and antigen-specific antibodies. These tests may be classified into two main groups: laboratory based-tests and point-of-care (POC) tests. Laboratory-based tests are sensitive and accurate, but require a laboratory setting and skilled technicians. POC tests are designed to be used in the field and require limited training, but they are far less sensitive and accurate with most POC tests providing only binary positive/negative or semi-quantitative results.

All current immunoassay technologies involve the formation of an antigen-antibody complex. The detection of the complex indicates the presence of a targeted analyte in a sample. The antigen-antibody complex is detected by measuring the emission and/or reflection of light by the complex, when fluorescent-tagged antigen-specific antibodies are employed, or in the case in which antibody-coated micro-beads are used, by measuring the emission and/or reflection of light, or the magnetic moment of the micro-beads forming the antigen-bead complex. In all cases, optical or magnetic detectors and electronic readers are required.

For example, the simplest, best known and widely used POC diagnostic assay is the lateral flow assay, also known as the immunochromatic test. In this test, the targeted analyte is bound to an analyte-specific antibody linked to latex or gold nanoparticles. The presence of the analyte in the sample then is revealed by the formation of a visible band, or line, which results from the agglutination or accumulation of the analyte-antibody-linked complex. The band typically is visible macroscopically to the naked eye. Devices to increase the assay's sensitivity have been developed which can read color changes with microscopic sensitivity. Fluorescent or magnetic-labeled particles also have been used. In these cases, however, electronic readers to assess test results are needed. Thus, although sensitivity of the assay may increase, the cost and complexity of the assay also increases.

In recent years, antibody-coated micro-beads have been increasingly used for the separation and detection of proteins. In the field of immunoassay diagnostics, the concentration of micro-beads is a proxy for the concentration of targeted proteins in a sample. In these applications, it is necessary to identify the concentration of micro-beads in the sample solution. The micro-beads may be made magnetically responsive by adding a magnetic core or layer to a polymer bead. The micro-beads then may be coated with a variety of molecules and proteins, referred to as ligands, which serve the purpose of binding the targeted antigen via an antibody-antigen interaction. In addition, fluorescent dyes can be incorporated into the micro-beads making them optically detectable. Recently, a diagnostic test for the protein troponin using magnetic micro-beads has been proposed by Dittmer et al. (Philips Research Europe). In this assay, micro-beads coated with anti-troponin antibody are immobilized via antibody-troponin-antibodies on the surface of a micro-well with the aid of an applied magnetic field. The number of antibody-troponin-antibodies is measured by illuminating the bottom of the well and measuring the light reflected by the immobilized micro-beads with an optical receiver. Methods for the detection of *E.coli* also have been developed using immuno-magnetic micro-beads. In this case, the bacteria in the sample are measured by detecting time-resolve fluorescence.

While micro-bead technology has matured in the last decade, the technology to quantify micro-bead concentration has lagged behind. Current methods include manual microscopes and automatic or semi-automatic cell counters. Typically, micro-bead counting using a microscope involves the manual, and often tedious, counting of beads through a microscope objective. This method requires skilled technicians in a laboratory setting, is time consuming and is subject to a technician's interpretation. Cell counters require photo sensors to detect micro-beads automatically by measuring the light reflection of a laser beam hitting the micro-bead's surface. Cell counters, while accurate, are expensive and also require skilled technicians in sophisticated laboratory settings. Lab-on-a-chip devices to detect and measure the concentration of protein-coated micro-bead concentration also have been developed. These devices, however, rely on traditional approaches, i.e., light reflection and detection using micro-scale light and photo sensors and micro-scale magneto-resistance magnetometers. Thus, while greatly reducing the need for a laboratory setting and equipment, lab-on-a-chip devices still require electrical readers and transducers. In addition, these devices typically include handset and consumable components, resulting in increased manufacturing, calibration and maintenance costs. Thus, these devices have limited applications in the field of POC immunoassay diagnostics.

There exists a need, therefore, for a POC immunoassay device which has the sensitivity and specificity of laboratory-based immunoassay tests while being simple to use and low cost, as well as for methods to detect and quantify magnetic-responsive micro-bead concentration in a sample specimen.

SUMMARY OF THE INVENTION

The *pScreen™* microfluidic immunoassay device, based on the inventions disclosed herein, fulfills all of the above-described needs in a single device. The detection and quantification of an unknown concentration of analyte in a liquid sample is obtained by exploiting the fluid-dynamic properties of magnetic-responsive micro-beads in liquid solution rather than using optical effects or magnetic field sensing as in current technologies. The unknown concentration of the target analyte is derived by measuring the differential flow rate between the sample flow in two micro-channels, one of which is under the influence of an applied magnetic field gradient. The present invention significantly reduces the cost and complexity of current laboratory-based immunoassay diagnostic tests, and greatly increases one-thousand fold the sensitivity of lateral flow tests, while maintaining the specificity and accuracy of laboratory-based tests, and the ability to detect targeted antigen concentration over a predefined range.

In an embodiment of the present invention, there is provided a method of detecting and quantifying the concentration of magnetic-responsive micro-beads in a fluid. The method comprises measuring flow rate *(Qm)* of a fluid in at least one test micro-channel *(Cm)* exposed to a magnetic field gradient with flow rate *(Qo)* of the fluid in a calibration micro-channel (*Co*) not exposed to a magnetic field gradient, in which the micro-channels are kept at an equal and constant pressure, and then calculating the ratio *Qm/Qo*, the difference *Qo-Qm,* and the ratio *(Qo-Qm)$^p$/(Qm)$^q$*, wherein *p and q* are derived through a calibration process, and wherein the ratios *Qm/Qo* and *(Qo-Qm)$^p$/(Qm)$^q$* are a proxy for the number of magnetic-responsive micro-beads in the fluid. The presence of magnetic-responsive micro-beads in the at least one test micro-channel which is exposed to the magnetic field gradient causes flocculation of the magnetic-responsive micro-beads in the fluid which reduces the flow rate of the fluid through the at least one test micro-channel.

In another embodiment, there is provided a method for detecting and quantifying concentration of an analyte in a liquid sample. The method comprises adding a liquid sample to a liquid sample inlet of a reaction chamber. The reaction chamber has adsorbed on its surface a plurality of immobilized antigen-specific antibodies (Ab1) specific to an analyte. The surface of the reaction chamber also has a plurality of magnetic-responsive micro-beads desiccated thereon, in which each of the plurality of magnetic-responsive micro-beads is coated with an antigen-specific antibody (Ab2) specific to the analyte. The method comprises having the liquid sample incubate within the reaction chamber, which causes rehydration of the plurality of antibody-coated magnetic-responsive micro-beads as the liquid sample is added and agitated in the reaction chamber, which rehydration disperses the antibody-coated magnetic-responsive micro-beads in the liquid sample, binding the rehydrated antibody-coated magnetic-responsive micro-beads as well as the antigen-specific antibodies immobilized on the surface of the reaction chamber to any analyte present in the liquid sample to form Ab1-analyte-Ab2-coated magnetic micro-bead complexes on the surface of the reaction chamber, having the liquid sample containing any unbound antibody-coated magnetic-responsive micro-beads exit the reaction chamber through a chamber outlet and transfer through a continuous fluid connection to a micro-channel splitter which bifurcates to form a calibration micro-channel (*Co*) and at least one test micro-channel *(Cm).* The at least one test micro-channel and the calibration micro-channel are kept at an equal and constant pressure. The calibration micro-channel is in continuous fluid connection with a graduated column, and the at least one test micro-channel is in continuous fluid connection with at least one graduated column. Each of the graduated columns has a graduated scale thereon. The method comprises measuring flow rate *(Qm)* of the liquid sample in the at least one test micro-channel exposed to a magnetic field gradient with flow rate *(Qo)* of the fluid in the calibration micro-channel not exposed to a magnetic field gradient, in which the presence of any unbound antibody-coated magnetic-responsive micro-beads in the at least one test micro-channel which is exposed to the magnetic field gradient causes flocculation of the antibody-coated magnetic-responsive micro-beads in the liquid sample which reduces the flow rate of the liquid sample through the at least one test micro-channel, then calculating the ratio *Qm/Qo*, the difference *Qo-Qm,* and the ratio *(Qo-Qm)$^p$/(Qm)$^q$*, wherein *p and q* are derived through a calibration process, and wherein the ratios *Qm/Qo* and *(Qo-Qm)$^p$/(Qm)$^q$* are a proxy for the number of magnetic-responsive micro-beads in the liquid sample, which is a proxy for the concentration of analyte in the liquid sample.

In a further embodiment of the present invention, there is provided a single use, portable, lab-on-card microfluidic *pScreen™* magnetic-responsive micro-bead concentration counter device for detecting and quantifying the concentration of magnetic-responsive micro-beads in a liquid sample. The microfluidic device is comprised of a liquid sample inlet defined by an opening for accepting a liquid sample that contains a quantity of magnetic-responsive micro-beads. The liquid sample inlet is in continuous fluid connection with a flow resistor, which is in continuous fluid connection with a micro-channel splitter which bifurcates to form a calibration micro-channel (*Co*) and at least one test micro-channel *(Cm).* The calibration micro-channel and the at least one test micro-channel are kept at an equal and constant pressure. The calibration micro-channel is in continuous fluid connection with a graduated column, and the at least one test micro-channel is in continuous fluid connection with at least one graduated column. The at least one test micro-channel is exposed to a magnetic field gradient, which causes flocculation of the magnetic-responsive micro-beads in the at least one test micro-channel. The flocculation reduces the flow rate *(Qm)* of the liquid sample in the at least one test micro-channel compared to the flow rate *(Qo)* of the liquid sample in the calibration micro-channel. Each of the graduated columns has a graduated scale thereon which provides a read-out of the total volume of the liquid sample collected in each of the graduated columns, in which the total volume of the liquid sample collected in the at least one test micro-channel graduated column indicates the concentration of magnetic-responsive micro-beads in the liquid sample.

In yet another embodiment of the invention, there is provided a single use, portable, lab-on-card microfluidic *pScreen™* immunoassay device for detecting and measuring an analyte in a liquid sample. The microfluidic immunoassay device is an assembly of the microfluidic *pScreen™* device described above and an immunoassay reaction chamber.

In particular, the microfluidic *pScreen™* immunoassay device comprises a liquid sample inlet defined by an opening for accepting the liquid sample. The liquid sample inlet is in continuous fluid connection with a flow resistor channel, which is in continuous fluid connection with an assay inlet of a reaction chamber. The reaction chamber has adsorbed on its surface a plurality of immobilized antigen-specific antibodies (Ab1) specific to an analyte, as well as having a plurality of magnetic-responsive micro-beads desiccated thereon. Each of the plurality of magnetic-responsive micro-beads is coated with an antigen-specific antibody (Ab2) specific to the analyte. Flow of the liquid sample through the reaction chamber rehydrates the plurality of antibody-coated magnetic-responsive micro-beads which disperses into the liquid sample. Any analyte present in the liquid sample binds to the dispersed antibody-coated magnetic-responsive micro-beads as well as to the antigen-specific antibodies immobilized on the surface of the reaction chamber to form Ab1-analyte-Ab2-coated magnetic-responsive micro-bead complexes. Any unbound antibody-coated magnetic-responsive micro-beads exit the reaction chamber through an assay outlet, which is in continuous fluid connection with a micro-channel splitter that bifurcates to form a calibration micro-channel *(Co)* and at least one test micro-channel *(Cm),* which are kept at an equal and constant pressure. The calibration micro-channel is in continuous fluid connection with a graduated column, and the at least one test micro-channel in continuous fluid connection with at least one graduated column. The at least one test micro-channel is exposed to a magnetic field gradient, which causes flocculation of the magnetic-responsive micro-beads in the at least one test micro-channel. The flocculation reduces the flow rate $(Qm)$ of the liquid sample in the at least one test micro-channel compared to the flow rate *(Qo)* of the liquid sample in the calibration micro-channel. Each of the graduated columns has a graduated scale thereon which provides a read-out of the total sample volume collected in each of the graduated columns, in which the total sample volume collected in the at least one test micro-channel graduated column indicates the concentration of analyte in the liquid sample.

As described above, the ratios $Qm/Qo$ and $(Qo-Qm)^p/(Qm)^q$ are a proxy for the number of magnetic-responsive micro-beads in a liquid sample, which is a proxy for the concentration of analyte in the liquid sample.

The devices may be fabricated by methods which include, without limitation, etching each of the micro-channels on a plastic substrate using a laser etcher system and then sealing the top of each of the micro-channels in plastic by thermal bonding, and by injection mold casting in plastic. Suitable plastic substrates, plastic sealing and injection mold casting plastics include, without limitation, poly(ethylene terephthalate) glycol, poly(lactic-co-glycolic acid) and poly(methyl methacrylate), respectively.

Liquid samples that can be assayed in accordance with the embodiments of the invention include, without limitation, water, plasma, serum, buffer solution, urine, whole blood, blood analogs, and liquid solutions from dilution of solid biological matter or other biological fluids.

Analytes that can be detected and quantified in accordance with the embodiments of the invention include, without limitation, proteins, protein fragments, antigens, antibodies, antibody fragments, peptides, RNA, RNA fragments, functionalized magnetic micro-beads specific to $CD^{4+}$, $CD^{8+}$ cells, malaria-infected red blood cells, cancer cells, cancer biomarkers such as prostate specific antigen and other cancer biomarkers, viruses, bacteria such as *E. coli* or other pathogenic agents.

The magnetic field gradient in accordance with the invention is generated from two magnets aligned lengthwise with the at least one test micro-channel and along opposite poles to expose the at least one test micro-channel to the magnetic field gradient. The at least one test micro-channel is located between a gap formed between the opposite poles of the magnets. In another embodiment, the magnetic field gradient is generated by one magnet and a magnetic-responsive structure positioned near the at least one test micro-channel.

In accordance with the invention, the magnetic field generated can range between about 0.05 Tesla (T) to about 0.5 T, and the magnetic field gradient that is generated can be about 10T/m or greater.

The total sample volume collected in the calibration micro-channel graduated column serves as a control for parameters such as variation in viscosity between samples, level of hematocrit in blood samples, temperature and humidity fluctuations and sample volumes.

In one embodiment of the invention, the micro-channel splitter of the microfluidic devices bifurcates to form one test micro-channel and one calibration micro-channel.

In another embodiment of the invention, the micro-channel splitter of the microfluidic device bifurcates to form three test micro-channels and one calibration micro-channel, in which each of the three test micro-channels is in continuous fluid connection with one graduated column.

In another embodiment of the invention, the micro-channel splitter of the microfluidic device bifurcates to form four test micro-channels and one calibration micro-channel, in which the four test micro-channels merge to be in continuous fluid connection with one graduated column.

The present invention will be more fully understood from the following description of the invention and by reference to the figures and claims appended hereto.

BRIEF DESCRIPTION OF THE DRAWINGS

A fuller understanding of the invention can be gained from the following description when read in conjunction with the accompanying drawings in which:

FIG. 1 is a schematic illustration of the method for determining the number of magnetic-responsive micro-beads in a fluid, in which the ratio between the flow rate in the calibration micro-channel *(Co)* and the test micro-channel *(Cm)* is measured, according to the embodiments of the invention;

FIG. 2 is a schematic illustration of the microfluidic *pScreen™* magnetic-responsive micro-bead concentration counter device, having one test micro-channel and one calibration micro-channel, according to the embodiments of the invention;

FIG. 3 is an artistic rendering of the microfluidic *pScreen™* immunoassay device, according to the embodiments of the invention;

FIG. 4 is a schematic illustration of the microfluidic *pScreen™* magnetic-responsive micro-bead concentration counter device, having three test micro-channels and one calibration micro-channel, according to the embodiments of the invention;

FIG. 5 is a schematic illustration of the microfluidic *pScreen™* magnetic-responsive micro-bead concentration counter device, having four test micro-channels and one calibration micro-channel, according to the embodiments of the invention;

FIG. 6 is the schematic illustration of the method for determining the concentration of analyte in a fluid, in which the sample analyte is bound to immobilized analyte-specific immobilized antibodies and to analyte-specific coated magnetic-responsive micro-beads, the ratio $Q_m/Q_o$, of the flow rate $Q_o$ in the calibration micro-channel, Co, and the flow rate, $Q_m$, in the test micro-channel, *Cm,* is measured, according to the embodiments of the invention.

FIG. 7 is a schematic illustration of the *pScreen™* immunoassay device, according to the embodiments of the invention;

FIG. 8 is a schematic illustration of three different views of a reaction chamber of the *pScreen™* immunoassay device, in which (A) shows the secondary antibody (Ab2)-coated magnetic-responsive micro-beads and primary antibody (Ab1)-capturing antibodies on the surface of the reaction chamber; (B) shows the Ab1-antigen-Ab2-magnetic-responsive micro-bead complexes immobilized on the surface of the chamber; and (C) shows unbound, i.e., free, Ab2-magnetic-responsive micro-beads reaching the assay outlet of the reaction chamber, according to the embodiments of the invention;

FIG. 9 is a schematic illustration which shows the formation of the Ab1-antigen-Ab2-magnetic-responsive micro-bead complexes as the sample with the antigen flows through the reaction chamber and rehydrates the magnetic-responsive micro-beads, according to the embodiments of the invention;

FIG. 10 is an artistic rendering of the *pScreen™* immunoassay device, according to the embodiments of the invention;

FIG. 11 is a graph showing reduction in fluid flow rate , i.e., the ratio between flow rate in the test (with magnetic field) and calibration (without magnetic field) micro-channels versus the number of magnetic-responsive micro-beads in the flocculation region;

FIG. 12 is a photomicrograph showing magnetically-induced flocculation of magnetic-responsive micro-beads at a concentration of about 2,000 micro-beads/$\mu$l in a test micro-channel, according to the embodiments of the invention; the inset shows the variation in flocculation at 300 seconds, 600 seconds and 800 seconds;

FIG. 13 is a graph showing the concentration of magnetic-responsive micro-beads in a solution obtained using the *pScreen*™ device versus the nominal concentration as tested by standard hemocytometry; and

FIG. 14 is a graph showing the concentration of immunoglobulin (IgG) in a solution obtained using the *pScreen*™ immunoassay.

DETAILED DESCRIPTION OF THE INVENTION

As used herein, the terms "magnetic-responsive micro-beads," "magnetic micro-beads" and "micro-beads" are meant to be interchangeable.

As used herein, the terms "analyte" and "antigen" are meant to be interchangeable.

As used herein, the terms "calibration micro-channel(s)" and "control micro-channel(s)" are meant to be interchangeable.

The present invention provides a flow rate-based method for detecting and quantifying the concentration, i.e., number, of magnetic-responsive micro-beads in a fluid. The ratio, *Qm/Qo*, between the flow rate *(Qm)* in a test micro-channel *(Cm)* exposed to a localized high-gradient magnetic field, and the unperturbed flow rate *(Qo)* in a calibration, or control, micro-channel *(Co)* not exposed to the localized high-gradient magnetic field, is a monotonic function of the number of micro-beads flowing through the test micro-channel. That is:

$$Qm(N_m)/Qc = f(N_m) \qquad \text{Equation (1)}$$

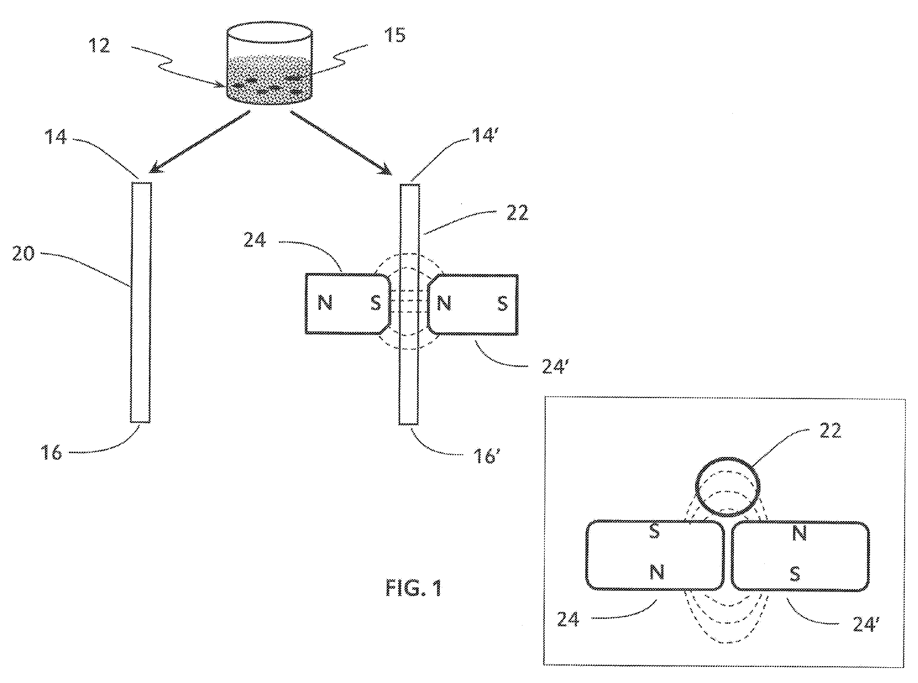

where *Nm* is the total number of magnetic-responsive micro-beads transported by the fluid into the localized high-magnetic field region. Both micro-channels are held at an equal and constant pressure. As shown in FIG. 1, a fluid 12 seeded with magnetic-responsive micro-beads 15 flows into two micro-channel inlets 14, 14' and out of two micro-channel outlets 16, 16'. The test micro-channel 22 on the right is exposed to a high-gradient magnetic field generated by two magnets 24, 24'. The magnets are positioned as shown in FIG. 1 and in the inset. In an embodiment, the magnetic field gradient is generated by one magnet and a magnetic-responsive structure (not shown) positioned near the test micro-channel 22. A magnetic-responsive structure may be made of a metallic material with ferromagnetic, super-paramagnetic or paramagnetic properties, such that upon application of an external magnetic field the magnetic-responsive structure generates an induced magnetic field. The structure is geometrically shaped, e.g., cylindrically-shaped, in order to generate a magnetic field gradient in the region occupied by the test micro-channel. Equation 1 applies to a wide range of magnetic-responsive micro-bead concentrations, ranging from about 50 micro-beads/$\mu$l to about 2 x 10$^6$ micro-beads/$\mu$l. The upper and lower limits, however, are a function of the micro-channels' size and magnetic field topology. Hence, both upper and lower limits may vary based on these parameters.

*Because f(Nm)* is a monotonic function of *Nm,* it also holds that:

$$N_m = f^{-1}(Qm(N_m)/Qc). \qquad \text{Equation (2)}$$

Thus, according to Equation 2, the number of magnetic-responsive micro-beads in a given fluid is a monotonic function of the ratio *Qm/Qo*. Thus, the number of magnetic-responsive micro-beads can be determined by measuring the ratio *Qm/Qo* in the two micro-channels, configured as shown in FIG. 1. In other words, the ratio *Qm/Qo* is a specific proxy for the number of magnetic-responsive micro-beads in a given fluid.

The analytical form of the function depends on the geometry, i.e., length and inner diameter of the two micro-channels, magnetic field topology, and the size of the magnetic-responsive micro-beads. In addition, the difference $Q_o-Q_m$, and the ratio $(Qo-Qm)^p/(Qm)^q$, where *p* and *q* are derived through a calibration process, are a proxy for the number of magnetic-responsive micro-beads in the fluid. The parameters *p* and *q* are obtained as followed. A solution containing a known concentration of micro-beads and of known volume is passed through the micro-channels and the flow rate *Qm* and *Qo* are measured. Then, a solution containing the same concentration of magnetic-responsive micro-beads but of larger volume similarly is passed through the micro-channels. This process is repeated several times. Then, the ratio $(Qo-Qm)^p/(Qm)^q$, with *p* and *q* set equal to 1, are plotted versus the volume of each sample. Using mathematical optimization methods, *p* and *q* are determined by enforcing the condition that the ratios $(Qo-Qm)^p/(Qm)^q$ versus sample volume form a horizontal straight line with slope equal to zero.

The present invention further provides a microfluidic *pScreen*™ magnetic-responsive micro-bead concentration counter device for detecting and quantifying magnetic-responsive micro-bead concentration in a liquid sample. This device leverages the previously described flow rate-based detection and quantification method.

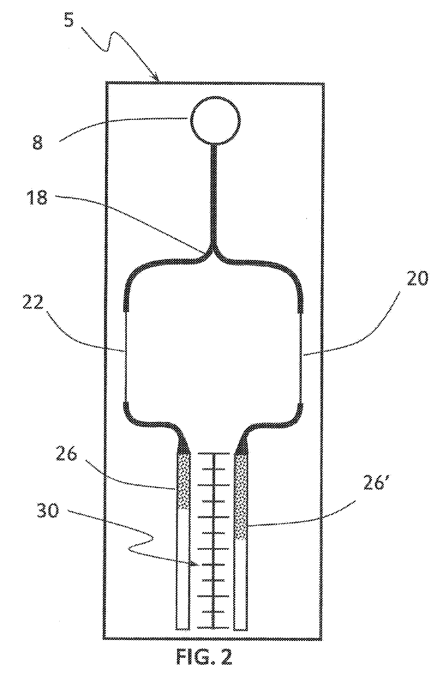

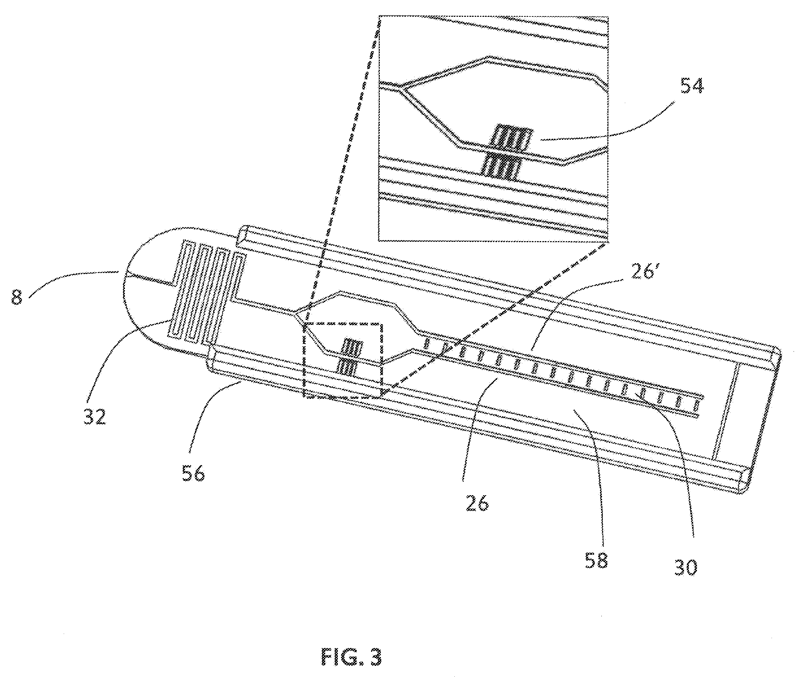

FIGS. 2 and 3 show the *pScreen™* microfluidic device 5 for the detection and quantification of magnetic-responsive magnetic beads in a liquid sample of the present invention. The microfluidic device 5 comprises a liquid sample inlet 8 in which a liquid sample, or specimen, which contains an unknown amount of magnetic-responsive micro-beads, is applied. From the liquid sample inlet 8, the liquid sample, self-propelled by capillary action, flows through a flow resistor 32 (shown in FIG. 3) and enters a micro-channel splitter 18. The micro-channel splitter 18 bifurcates into two smaller micro-channels: a calibration micro-channel 20 and a test micro-channel 22. The two micro-channels 20, 22 are identical in length and inner diameter (best shown in FIG. 2).

The concentration of magnetic-responsive micro-beads that can be detected and quantified using the methods and devices of the invention is about 50 micro-beads/$\mu$l to about $2 \times 10^6$ micro-beads/$\mu$l; and the diameter of the magnetic-responsive micro-beads is about 0.2 $\mu$m to about 20 $\mu$m. In an embodiment, the diameter of the magnetic micro-beads is about 4.0 $\mu$m.

In accordance with the invention, the test micro-channel and the calibration micro-channel are made of a capillary tube, in which the length of the capillary tube is about 0.2 cm to about 20 cm. In an embodiment, the length of the capillary tube is about 3.0 cm to about 7.5 cm. In another embodiment, the length of the capillary tube is about 1.5 cm.

In an embodiment, the length of the calibration micro-channel 20 and the test micro-channel 22 is about 0.2 cm to about 20 cm. In another embodiment, the length of the two micro-channels 20, 22 is about 3.0 cm to about 7.5 cm. In still another embodiment, the length of the two micro-channels 20, 22 is about 1.5 cm.

In an embodiment, the inner diameter of the calibration micro-channel 20 and the test micro-channel 22 is about 50 $\mu$m to about 500 $\mu$m. In another embodiment, the inner diameter of the two micro-channels 20, 22 is about 50 $\mu$m.

A magnetic field gradient is applied only to the test micro-channel 22. The magnetic field gradient is generated by small rare-earth (e.g., neodymium) permanent magnet and ferromagnetic (e.g., nickel, iron) pole structures (not shown) which serve as a magnetic concentrator 54 (shown in FIG. 3) specifically designed to concentrate the magnetic field, hence creating a high magnetic field gradient (of about 100 T/m). In the calibration channel 20, the liquid sample flows freely at a very low velocity (Reynolds number around 1) and shear rate range (1 to 400s$^{-1}$). In the test micro-channel 22, the magnetic field gradient induces micro-bead flocculation if magnetic-responsive micro-beads are present in the sample. Even in very small concentrations, as low as < 50 micro-beads/$\mu$l, the flow rate through the test micro-channel 22 will be reduced due to the formation of the magnetically-induced micro-bead flocculation. After flowing through the calibration and test micro-channels 20, 22, a volume of liquid sample is collected in two graduated columns 26, 26', both of equal size and volume.

Each graduated column 26, 26' has a graduated scale thereon 30 which provides an easy to interpret read-out system of the total sample volume collected in each graduated column 26, 26'. The graduated columns' 26, 26' length and cross section, as well as the respective scales 30 thereon, are designed to be visible to the naked eye. Unlike current POC read-out devices, the read-out system of the microfluidic device of the present invention does not require electrical transducers and/or sensors. As shown in FIGS. 2 and 3, both graduated columns 26, 26' are clearly visible. As shown in FIG. 3, the microfluidic device 5 may be configured in a cartridge 58 which fits into a holder 56. The read-out obtained by the microfluidic device 5 can be determined at any time by direct comparison of the fluid in the two graduated columns 26, 26'.

Referring now to FIG. 2, the micro-channel configuration provides for the concentration of micro-beads to be a monotonic function only of the volumes *Vo* and *Vm* (*Vo* and *Vm* are the volumes collected at the micro-channel outlets 16, 16' of the calibration micro-channel 20 *(Co)* and test micro-channel 22 *(Cm),* respectively; shown in FIG. 1). This approach allows the user to read out the result provided by the *pScreen™* microfluidic device of the present invention at any time while the assay is running or at any time after the assay has been completed.

Given the relationship in Equation (1), and because the flow rate in the calibration micro-channel 20 is constant and the magnetic-responsive micro-beads are uniformly distributed in the sample fluid, the below identities are satisfied at any time instances:

$$T = \int_0^{N_0} \frac{dN'}{\rho Q_0} = \frac{N_0}{\rho Q_0} \, , \qquad\qquad\qquad \text{Equation (3)}$$

$$T = \int_0^{N_m} \frac{dN'}{\rho Q_m} \, . \qquad\qquad\qquad \text{Equation (4)}$$

where $\rho$ is the magnetic-responsive micro-bead concentration in the sample specimen 12, *Qo* and *Qm* are the flow rates in the calibration and test micro-channels 20, 22, respectively, and *No* and *Nm* is the number of magnetic-responsive micro-beads passing through the calibration and test micro-channels 20, 22, respectively.

It thus follows that:

$$N_0 = g(N_m), with : g(N) \equiv \int_0^{N_m} \frac{dN'}{\hat{Q}(N)}, and : \hat{Q} = \frac{Q_m}{Q_0} .$$

Equation (5)

Thus: $N_m = g^{-1}(N_0)$.

Equation (6)

Since, $N_0 = \rho V_0$, and $N_m = \rho V_m$, we have that: $\rho V_m = g^{-1}(\rho V_0)$.

Equation (7)

Hence: $\rho = F(V_0, V_m)$.

Equation (8)

Thus, the *pScreen*™ microfluidic device of the present invention provides a comparative read-out system in which the magnetic-responsive micro-bead concentration, ρ, is a monotonic function of only *Vm*, the volume flowing through the test micro-channel 22 where the magnetic-induced flocculation forms and *Vo*, the volume flowing through the calibration micro-channel 20 without the magnetic-induced flocculation.

The comparative read-out system of the *pScreen*™ microfluidic device of the present invention greatly simplifies the detection and quantification of magnetic-responsive micro-bead concentration in a liquid sample. In addition, this comparative read-out system has the significant advantage of virtually eliminating common-mode error (with the calibration graduated column 26 acting as a control), such as variation in viscosity between samples, level of hematocrit in blood samples, temperature and humidity fluctuation of the test environment, and sample volume. The *pScreen*™ microfluidic device of the present invention thus provides a stand-alone device for the detection and quantification of magnetic-responsive micro-bead concentration in liquid samples over a wide range of concentrations and micro-bead sizes.

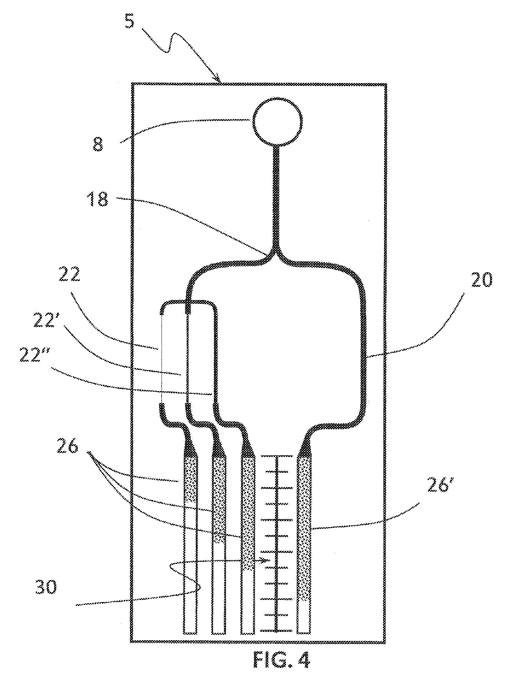

FIG. 4 shows an alternate embodiment of the microfluidic device 5 of the invention. In this embodiment, the test micro-channel (*Cm*) is split into three test micro-channels 22, 22' which 22" run parallel to one other. The three test micro-channels 22, 22' and 22" are of the same length but have a different inner diameter from one another. Each test micro-channel 22, 22', 22" is connected to a separate graduated column 26. In an embodiment, the first test micro-channel 22 has an inner diameter of about 50 μm to about 500 μm, the second test micro-channel 22' has an inner diameter of about 100 μm to about 250 μm, and the third test micro-channel 22" has an inner diameter of about 250 μm to about 5 mm. In another embodiment, the first test micro-channel 22 has an inner diameter of about 50 μm, the second test micro-channel 22' has an inner diameter of about 100 μm, and the third test micro-channel 22" has an inner diameter of about 250 μm. The inner diameter of the calibration micro-channel 20 is such that the area of the cross-section of the calibration micro-channel 20 is identical to the sum of the areas of the cross-sections of the three test micro-channels 22, 22', 22".

For a given amount of magnetic-responsive micro-beads entering each of the three test micro-channels 22, 22' 22", the third, largest test micro-channel 22" experiences the lowest reduction in flow rate, the second, middle-sized test micro-channel 22' experiences a reduction in flow rate greater than in the third, largest test micro-channel 22", and the first, smallest test micro-channel 22 experiences the greatest reduction in flow rate. In addition, the first, smallest test micro-channel 22 will tend to clog before the second, middle-sized test micro-channel 22' and the third, largest test micro-channel 22", and the middle-sized test micro-channel 22' will tend to clog before the largest test micro-channel 22". Hence, the device in accordance with this embodiment allows measurement of a wide range of concentrations of magnetic-responsive micro-beads, in which the first, smallest test micro-channel 22 allows for finely-tuned measurements of magnetic-responsive micro-beads at low concentrations and the third, largest test micro-channel 22" allows for gross measurements of magnetic-responsive micro-beads at high concentrations.

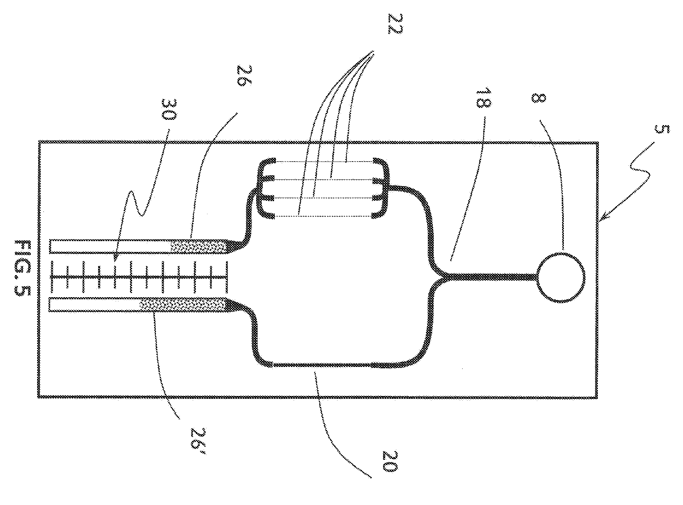

FIG. 5 shows an additional alternate embodiment of the invention. In this embodiment, the test micro-channel 22 (*Cm*) is split into four micro-channels which run parallel to each other. The four test micro-channels 22 have the same length and inner diameter. In an embodiment, each of the four test micro-channels has an inner diameter of about 12.5 μm to about 125 μm. In another embodiment, each of the four test micro-channels has an inner diameter of about 12.5 μm. The inner diameter of the calibration micro-channel 20 is such that the area of the cross-section of the calibration micro-channel 20 is identical to the sum of the areas of the cross-sections of the four test micro-channels 22, 22', 22".

The four test micro-channels 22 rejoin to connect to one graduated column 26. If no magnetic-responsive micro-beads flow into the four test-micro-channels 22 and the one calibration micro-channel 20, then the flow rate of the fluid through the calibration micro-channel 20 is the sum of the flow rates in each of the test micro-channels. Equations (1) through (8) also apply in this embodiment, however, because there are four parallel test micro-channels compared to one test micro-channel, a greater volume of fluid can flow through the device in a shorter amount of time, thus allowing a user to obtain a read out of results of the *pScreen*™ microfluidic device in a shorter period of time.

The present invention also provides a flow rate-based method for detecting and quantifying concentration of an analyte in a liquid sample. The analyte can include, without limitation, proteins, protein fragments, antigens, antibodies, antibody fragments, peptides, RNA, RNA fragments, cells, cancer cells, viruses, and other pathogenic agents.

The method according to this embodiment comprises adding a liquid sample to a liquid sample inlet of a reaction chamber. The reaction chamber has adsorbed on its surface a plurality of immobilized antigen-specific antibodies (Ab1) specific to an analyte. The surface of the reaction chamber also has a plurality of magnetic-responsive micro-beads desiccated thereon, in which each of the plurality of magnetic-responsive micro-beads is coated with an antigen-specific antibody (Ab2) specific to the analyte. The method comprises having the liquid sample incubate inside the reaction chamber, which causes rehydration of the plurality of antibody-coated magnetic-responsive micro-beads as the liquid sample is added and agitated in the reaction chamber, which rehydration disperses the antibody-coated magnetic-responsive micro-beads in the liquid sample, binding the rehydrated antibody-coated magnetic-responsive micro-beads as well as the antigen-specific antibodies immobilized on the surface of the reaction chamber to any analyte present in the liquid sample to form Ab1-analyte-Ab2-coated magnetic micro-bead complexes on the surface of the reaction chamber, having the liquid sample containing any unbound antibody-coated magnetic-responsive micro-beads exit the reaction chamber through a chamber outlet and transfer through a continuous fluid connection to a micro-channel splitter which bifurcates to form a calibration micro-channel (*Co*) and at least one test micro-channel (*Cm*). The at least one test micro-channel and the calibration micro-channel are kept at an equal and constant pressure. The calibration micro-channel is in continuous fluid connection with a graduated column, and the at least one test micro-channel is in continuous fluid connection with at least one graduated column. Each of the graduated columns has a graduated scale thereon. The method comprises measuring flow rate (*Qm*) of the liquid sample in the at least one test micro-channel exposed to a magnetic field gradient with flow rate *(Qo)* of the fluid in the calibration micro-channel not exposed to a magnetic field gradient, in which the presence of any unbound antibody-coated magnetic-responsive micro-beads in the at least one test micro-channel which is exposed to the magnetic field gradient causes flocculation of the antibody-coated magnetic-responsive micro-beads in the liquid sample which reduces the flow rate of the liquid sample through the at least one test micro-channel, and calculating the ratio *Qm*/*Qo*, the difference *Qo-Qm*, and the ratio $(Qo-Qm)^p/(Qm)^q$, wherein *p* and *q* are derived through a calibration process, and wherein the ratios *Qm*/*Qo* and $(Qo-Qm)^p/(Qm)^q$ are a proxy for the number of magnetic-responsive micro-beads in the liquid sample, which is a proxy for the concentration of analyte in the liquid sample.

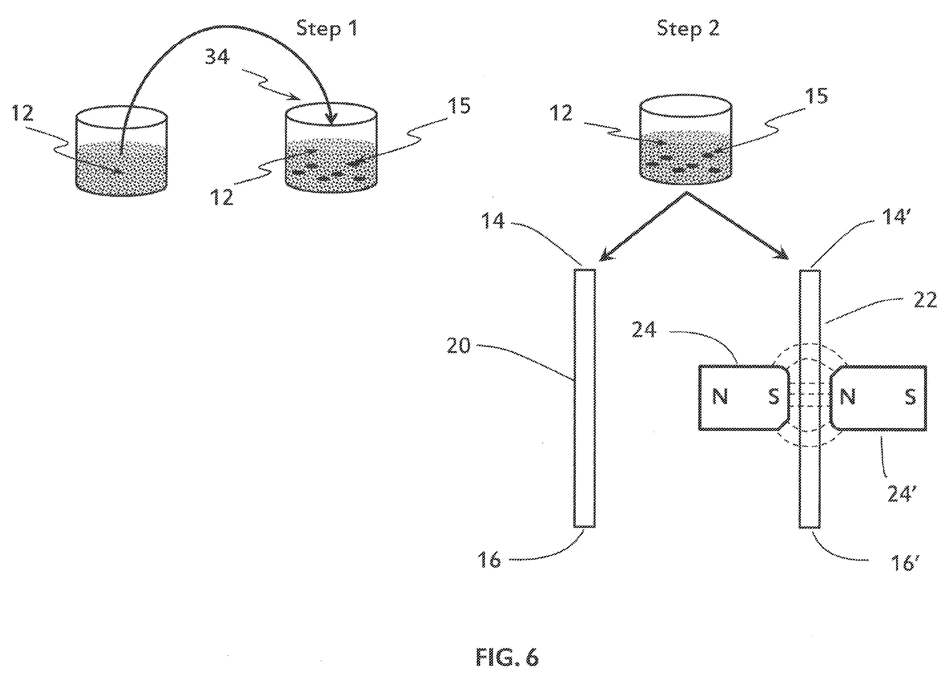

As shown in FIG. 6, a liquid sample 12 is added, Step 1, to a reaction chamber 34, which has adsorbed on its surface a plurality of immobilized antigen-specific antibodies (Ab1) (not shown) and contains a plurality of magnetic-responsive micro-beads coated with antigen-specific antibodies (Ab2) 15 desiccated on the surface of the reaction chamber 34. In Step 1, by adding the liquid sample to the reaction chamber 34, the antibody-coated magnetic-responsive micro-beads 15 are rehydrated and the magnetic-responsive micro-beads 15, as well as the antigen-specific antibodies immobilized on the surface of the reaction chamber 34, bind to any analyte present in the liquid sample 12 to form Ab1-analyte-Ab2-coated magnetic-responsive micro-bead complexes on the surface of the reaction chamber 34, with any unbound magnetic-responsive micro-beads 15 free to flow (Step 2) into two micro-channel inlets 14, 14' and out of two micro-channel outlets 16, 16'. The test micro-channel 22 on the right is exposed to a high-gradient magnetic field generated by two magnets 24. As described in the previous paragraph, the number of magnetic-responsive micro-beads in a liquid sample passing through two micro-channels, a test micro-channel *(Co)* and a calibration, or control micro-channel *(Cm)*, is proportional to the ratio, $Q_m/Qo$, between the flow rate (*Qm*) in a micro-channel (*Cm*) exposed to a localized high-gradient magnetic field, and the unperturbed flow rate *(Qo)* in a micro-channel *(Co)* not exposed to the localized high-gradient magnetic field. Therefore, by measuring the flow rates *Qm* and *Qo,* the concentration of analyte in the liquid sample can be determined. The method applies to a wide range of antigen concentration, from about 0.01 ng/ml to about 1 μg/ml.

The present invention further provides a *pScreen*™ microfluidic immunoassay device for the detection and quantification of proteins, protein fragments, antigens, antibodies, antibody fragments, RNA, RNA fragments, cells, cancer cells, viruses, and other pathogenic agents. This device leverages the previously described method for detecting and quantifying concentration of an analyte in a liquid sample.

Principle of Operation

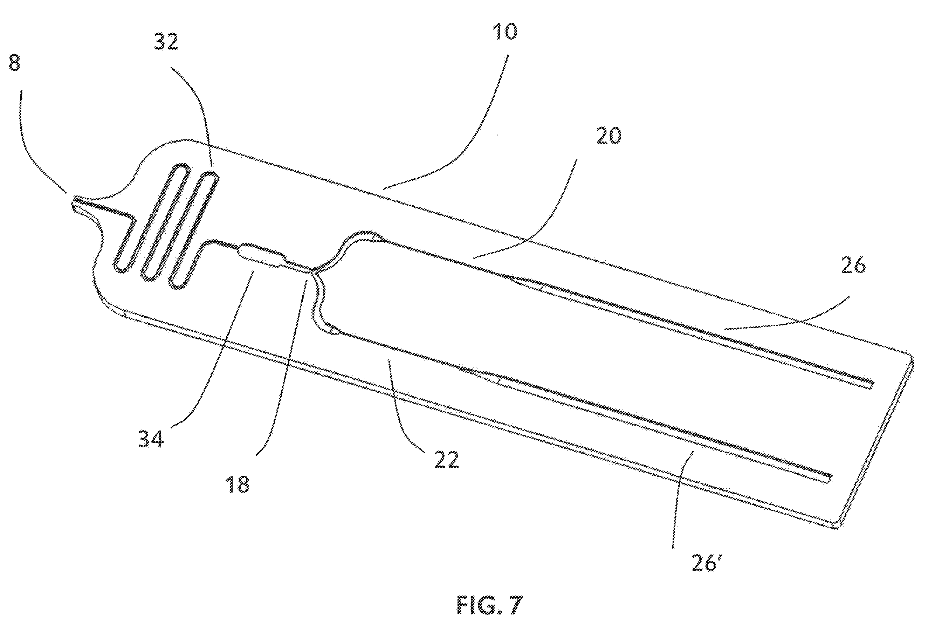

In one embodiment of the invention, as shown in FIG. 7, the *pScreen*™ microfluidic immunoassay device 10 has a liquid sample inlet 8, a flow resistor channel 32, a reaction chamber 34, a micro-channel splitter 18, a test micro-channel 22, a calibration micro-channel 20 and graduated readout columns 26, 26'. In use, a liquid sample, or specimen, which may contain an unknown amount of a target analyte, is applied into the liquid sample inlet 8. By capillary action, the sample is self-propelled and transferred from the liquid sample inlet 8 into the reaction chamber 34 via the flow resistor channel 32. The flow rate of the liquid sample is determined by the cross-section and length of the flow resistor channel 32 and the surface tension of the device material and sample liquid, as well as by the binding kinetic reaction between the analyte, i.e., antigen, and antibody in the reaction chamber 34.

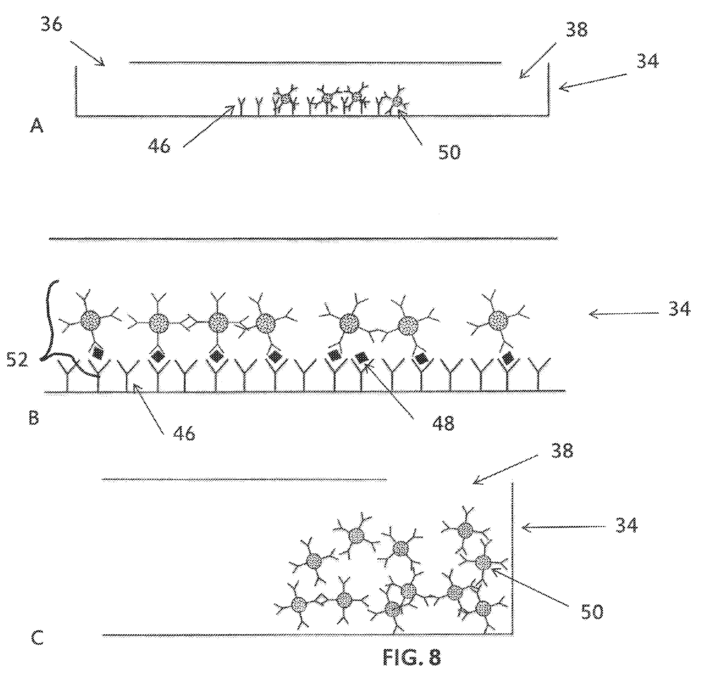

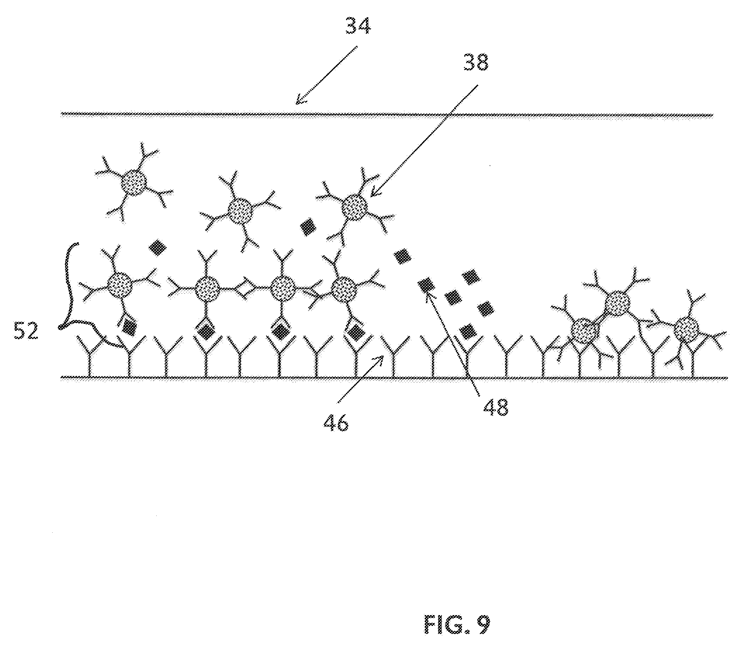

As shown in FIG. 8A, the reaction chamber 34 is coated with antigen-specific antibodies (Ab1) 46 immobilized onto the surface of the reaction chamber 34. The antigen-specific antibodies (Ab1) 46, referred to as capturing antibodies, may be primary or secondary antibodies. The antigen-specific antibodies (Ab1) 46 are bound to the surface of the reaction chamber 34 via adsorption. The surface of the reaction chamber 34 also is coated with magnetic-responsive micro-beads 50 by desiccation. The magnetic-responsive micro-beads may be desiccated on the same region of the device where Ab1 antibodies 46 are bound, or in a region preceding where the Ab1 antibodies 46 are bound. The magnetic-responsive micro-beads are coated with antigen-specific antibodies (Ab2) to form antigen-specific antibody-coated magnetic-responsive micro-beads 50. These antigen-specific antibodies (Ab2) may be primary or secondary antibodies. As the liquid sample flows into the reaction chamber 34 via an assay inlet 36, the antigen-specific antibody-coated magnetic-responsive micro-beads 50 are rehydrated and dispersed in the liquid, and any antigen molecules 48 contained in the sample bind to the antigen-specific antibodies (Ab1) 46 immobilized on the surface of the reaction chamber, and to the antigen-specific antibodies (Ab2) coating the magnetic beads, forming Ab1-antigen-Ab2-coated magnetic-responsive micro-bead complexes 52 (FIG. 8B). The formation of Ab1-antigen-Ab2-coated magnetic-responsive micro-bead complexes 52 anchors the bound antibody-coated magnetic-responsive micro-beads 50 onto the surface of the reaction chamber 34. After all antigen molecules 48 have reacted to form the Ab1-antigen-Ab2-coated magnetic-responsive micro-bead complexes 52, any unbound, i.e., free, antibody-coated magnetic-responsive micro-beads 50 exit the reaction chamber via an assay outlet 38 (FIG. 8C), leaving behind the bound antibody-coated magnetic-responsive micro-beads 50 in the reaction chamber 34. FIG. 9 shows the formation of the Ab1-antigen-Ab2-coated magnetic-responsive micro-bead complex 52 as a liquid sample containing an antigen 48 flows through the reaction chamber 34 and rehydrates the antibody-coated magnetic-responsive micro-beads 50.

A negative liquid sample, i.e., a sample not containing detectable traces of the targeted analyte, results in zero antibody-coated magnetic-responsive micro-beads anchored to the reaction chamber's surface, as the Ab1-antigen-Ab2-coated magnetic-responsive micro-bead complexes cannot form. An analyte (i.e.,antigen)-positive sample, on the other hand, results in antibody-coated magnetic-responsive micro-beads anchored to the reaction chamber's surface via the Ab1-antigen-Ab2-coated magnetic micro-bead complexes. Thus, the higher the concentration of analyte in the liquid sample, the greater the number of magnetic-responsive micro-beads anchored to the reaction chamber's surface, and hence the fewer the number of free magnetic-responsive micro-beads reaching the reaction chamber assay outlet. In the extreme case of very high analyte concentration, all antibody-coated magnetic-responsive micro-beads will be anchored to the reaction chamber's surface, and none will exit through the reaction chamber's assay outlet.

After flowing through the reaction chamber, the liquid sample, self-propelled by capillary action, reaches the *pScreen*™ magnetic bead concentration counter portion of the device (which principle of operation has been described previously). If the liquid sample flowing into the test micro-channel and the calibration micro-channel contains no magnetic-responsive micro-beads, the flow rate in both the test and calibration micro-channels will be identical, and thus the sample volume collected in each of the graduated columns will be identical. The user easily is able to observe that the volume of sample in each of the graduated columns is of equal length. On the other hand, if the sample coming from the micro-channel splitter contains magnetic-responsive micro-beads in any concentration other than zero, the flow of the liquid in the test micro-channel will be retarded (due to the magnetically-induced flocculation of the magnetic micro-beads). Hence, the length of the volume of liquid in the test graduated column will be less than the length of the volume of liquid in the calibration graduated column by an amount proportional to the magnetic-responsive micro-bead concentration in the volume of liquid flowing into the graduated columns. In other words, the higher the magnetic-responsive micro-bead concentration in the liquid reaching the test and calibration micro-channels, the greater the difference in the lengths of the volume of liquid observed in the two graduated columns. The resulting difference between the volumes of liquid collected in the two graduated columns is easily visible to the naked eye.

In an embodiment of the *pScreen*™ microfluidic immunoassay device, described in detail above and shown in FIG. 4, the test micro-channel (*Cm*) is split into three test micro-channels 22, 22' and 22" which run parallel to each other. In another embodiment of the *pScreen*™ microfluidic immunoassay device, described in detail above and shown in FIG. 5, the test micro-channel (*Cm*) is split into four micro-channels 22 which run parallel to each other.

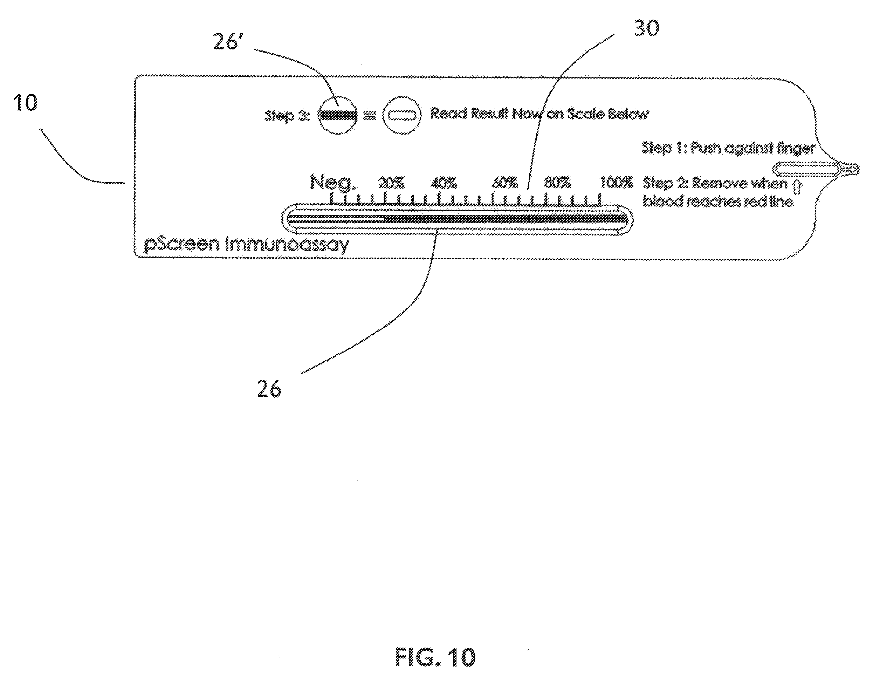

FIG. 10 shows the *pScreen*™ microfluidic immunoassay device 10, according to the embodiments of the invention, in which the calibration column is only partially visible. In this embodiment, the read-out is taken when the portion of the calibration column that is visible changes color, i.e., fills up with liquid.

EXAMPLES

The present invention is more particularly described in the following non-limiting examples, which are intended to be illustrative only, as numerous modifications and variations therein will be apparent to those skilled in the art.

Example 1 -Scientific Basis and Technology Feasibility of the Present Invention

(A) Introduction

The data presented herein describe the effect that flocculation of magnetic-responsive micro-beads in a micro-channel has on the flow resistance of liquid in the micro-channel. A liquid seeded with magnetic-responsive micro-beads in a micro-channel that is exposed to a magnetic field gradient produces a localized micro-bead flocculation. This localized micro-bead flocculation results in a localized reduction of the cross section of the micro-channel, and thus in a localized increase of the flow resistance across the flocculation region. The increase in resistance, in turn, results in an increased pressure drop across the flocculation region due to the energy loss in maintaining the flow across the reduced cross-section of the micro-channel. If the external forces responsible for the formation of the micro-bead flocculation are stronger than the flow shear-stress on the micro-beads and their aggregates, the micro-beads' flocculation increases in magnitude as more incoming micro-beads are added. In the case of a constant-pressure driven flow (relevant to the present invention), the increased pressure drop results in a reduction of the micro-channel flow rate. This study investigated and analyzed this phenomenon, and the results are reported below. These experimental results provide the scientific basis upon which the *pScreen*™ technology and the present invention have been developed.

(B) Experimental Methodology

Experimental data with respect to the effect of magnetic micro-bead flocculation on flow rate in micro-channels are presented. FIG. 1 is an illustration of a constant pressure flow system comprised of two micro-channels. Test micro-channel 22 (*Cm*) was exposed to a high-magnetic field gradient, while calibration micro-channel 20 *(Co)* served as a control. The pressure difference driving the flow between the micro-channel inlets 14, 14' and micro-channel outlets 16, 16' was equal between the two micro-channels 20, 22. A liquid sample seeded with a known concentration of magnetic-responsive micro-beads 12 was added to both micro-channels 20, 22 and the flow rate in both micro-channels 20, 22 was recorded over time. Flocculation of the micro-beads was created by means of a localized high-gradient magnetic field generated by two magnets 24, 24'.
Experiments were conducted using micro-channels fabricated from glass capillary tubes having an inner diameter of 50 μm and 100 μm. The length of the capillary tubes was varied between 3.0 cm and 7.5 cm. The magnetic field was generated by two neodymium-iron-boron (NdFeB) permanent magnets 24, 24' (25 mm × 6 mm × 1.5 mm; maximum surface field: 0.3 T). The magnets 24, 24' were aligned length-wise along opposite poles. The test micro-channel 22 capillary tube exposed to the magnetic field was placed between the gaps formed between the opposite poles of the NdFeB magnets 24, 24'.
Both micro-channel capillary tubes 20, 22 were partially inserted into a rubber stopper portion of a glass vacutainer tube (not shown), leaving about 0.5 cm of the ends of the capillary tubes visible. Each capillary tube inlet and outlet was inserted in a polystyrene tubing (not shown) having an inner diameter of 360 μm, which tightly fit the 360 μm outer diameter of the two micro-channel capillary tubes 20, 22. One end of the tubing lead directly to a reservoir containing the liquid sample with the magnetic micro-bead solution 12, and the other end of the tubing lead to the vacutainer tubes which collected the fluid exiting the micro-channel outlets 16, 16'. The sample reservoir 12 was open to the air, and thus was at atmosphere pressure. The vacutainer tubes were sealed and kept under a constant vacuum. The pressure difference between the sample reservoir 12 and the vacutainer tubes induced the liquid sample to flow from the reservoir into the vacutainer tubes. The pressure difference was maintained at 0.6 mmHg per cm of capillary tube to provide equal flow rate across capillary tubes of different lengths. Experiments were run in tandem, using two capillary tubes: one for the calibration, i.e., control, micro-channel 20; and one for the test micro-channel 22 exposed to the magnetic field gradient. Both micro-channel capillary tubes 20, 22 were kept at the same differential pressure and drew fluid from the same sample reservoir 12. In the calibration micro-channel capillary tube 20, the sample flowed freely. In the test micro-channel capillary tube 22, the applied magnetic field gradient induced micro-bead flocculation. The calibration and test micro-channels 20, 22 were run simultaneously to eliminate common error, such as variation in atmospheric pressure, changes in viscosity due to fluctuation in temperature, and variations in micro-bead concentration. The suspension medium was 35 % (by wt.) glycerol and 65 % water to achieve a viscosity similar to that of blood (about 3.6 cP). Green fluorescent dye was added to the suspension medium to increase visibility of the solution exiting the two micro-channel capillary tubes 20, 22. Also added to the medium were smooth carboxyl magnetic micro-beads (Spherotech, Inc.) with a diameter of 4.7 μm or 8.3 μm. Tests were conducted with a micro-bead concentration between 100 micro-beads/μl

to $50\times10^3$ micro-beads/µl. Sample volumes were between 50 µl to 200µl and initial flow rates were 0.01µl/sec. As the sample fluid exited the micro-channel capillary tubes 20, 22, it formed small drops before falling into the vacutainer. The measurement of flow rate was calculated by dividing the drop volume with the time interval between drops. The falling drop rate was recorded with a DVD video camera. Post video analysis provided the flow rate vs. time. Additional experiments were conducted without a vacutainer. The micro-channel outlets 16, 16' were connected to long polystyrene tubing (not shown) which was placed near a graduated ruler. The flow rate was measured by recording the advancement of the fluid meniscus inside the tubing as a function of time. Flow rate values in the glass calibration micro-channel capillary tube 20 not exposed to the magnetic field gradient were compared with theoretical Hagan - Poiseuille flow $Q = \pi\,R\,4\,\Delta P$ (wherein R is the tube radius, ΔP is the pressure $8\mu L$ difference, µ is the fluid viscosity, and L is the tube length) for a fully developed laminar flow of a Newtonian fluid in a cylindrical tube. Additional experiments were conducted using a micro-channel configuration as shown in FIG. 2 and fabricated in poly(lactic-co-glycolic acid) (PLGA) and Polyethylene terephthalate by laser etching as above described.

(C) Macroscopic Experimental Results and Data Analysis

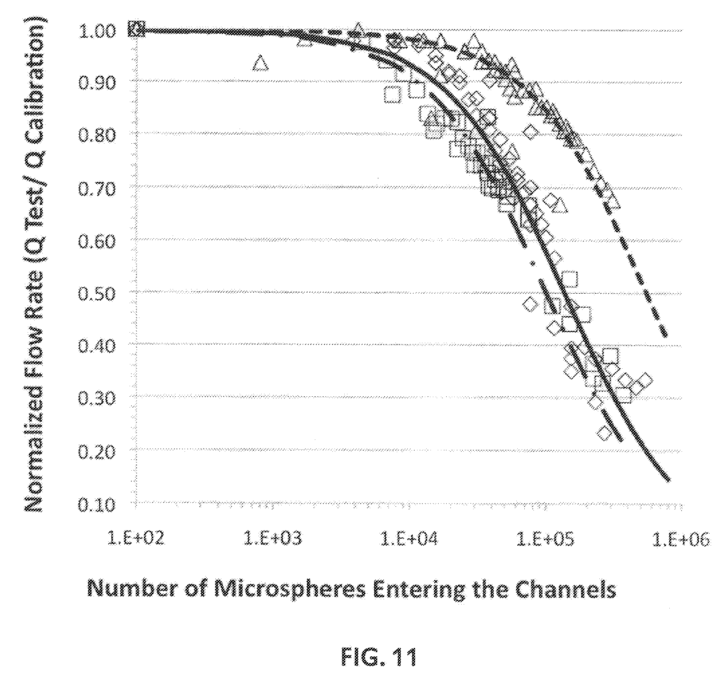

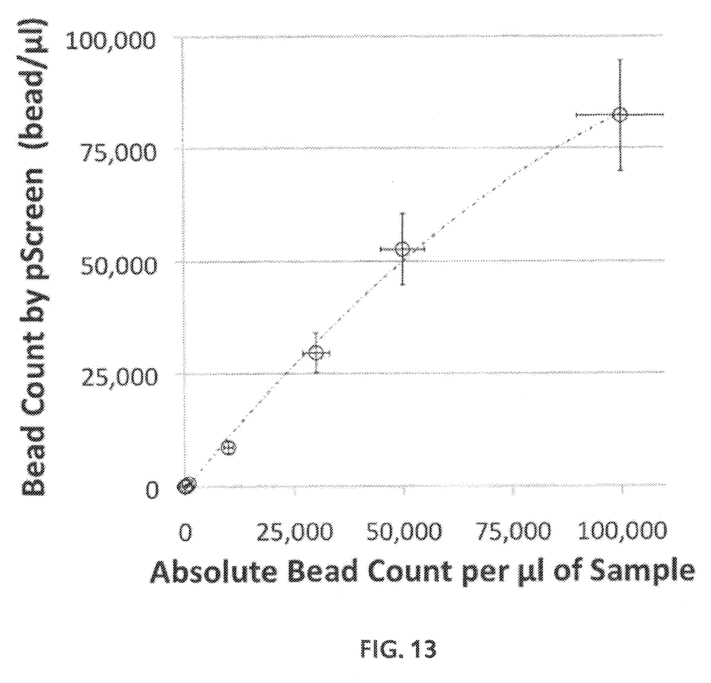

FIG. 11 shows the normalized flow rate, i.e., the ratio between the flow rate in the test micro-channel capillary tubes (exposed to the magnetic field gradient) and the calibration micro-channel capillary tubes (not exposed to the magnetic field gradient) versus the number of magnetic-responsive micro-beads in the flocculation region. The number of magnetic-responsive micro-beads is given by the product of the flow rate times the magnetic-responsive micro-bead concentration in the sample. The data show that the normalized flow rate is a monotonic function of the number (over three orders of magnitude) of magnetic-responsive micro-beads in the flocculation region. The amount by which the flow rate is reduced due to the pressure drop caused by the flocculation depends on the overall capillary length and the size of the flocculation zone. Thus, different aspect ratios of magnetic field length along the micro-channels versus the total length of the micro-channels also were investigated. FIG. 13 shows three data curves for different aspect ratios of magnetic field (concentrator) length versus the total length of the micro-channels [ratios range from 0.17 (triangle) to 0.24 (diamond) to 0.4 (square)]. To the investigators' knowledge, these data are the first to provide direct measurements of the effect of magnetic-responsive micro-bead flocculation on fluid flow rate in micro-channels.

(D) Data Analysis

These experimental data demonstrate that over a wide range the normalized flow rate, $Qm/Qo$, is a monotonic function of the number of magnetic-responsive micro-beads entering the capillary tubes. What is presented herein is a phenomenological model based on the Poiseuille equation that the investigators derived to corroborate these results. The model relates the flow rate to the reduction in micro-channel cross-section due to the formation of flocculation. The model predicts the following relationship between flow rate and number of magnetic-responsive micro-beads:

$$\hat{Q} = 1/(1+\alpha N), \qquad\qquad \text{Equation (9)}$$

$$\alpha = \frac{(1-a/R_{eff})^4}{LB}, \qquad\qquad \text{Equation (10)}$$

$$B = 3/4 \cdot (1-\varepsilon) \cdot \frac{(a^2 - R^2_{eff})}{r^3}. \qquad\qquad \text{Equation (11)}$$

where $Q$ is the normalized flow rate, $N$ is the number of micro-beads in the flocculation, $\alpha$ is the capillary tube radius, $R_{eff}$ is the lumen length of the capillary tube not blocked by micro-bead flocculation, $L$ is the capillary tube length, and r the radius of the micro-beads. The model predicts with high accuracy (solid lines, FIG. 11) the curve shift with changes in capillary length over magnetic field region lengths. This model provided analytical guidance for designing the specifications of the device of the present invention.

(E) Microscopic Experimental Results

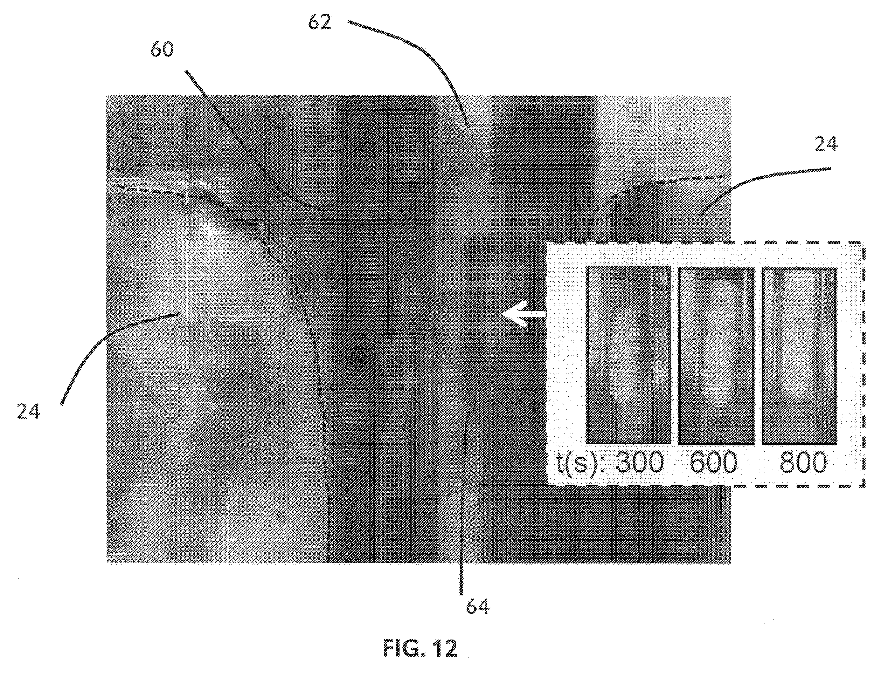

In order to observe the mechanism of magnetically-induced flocculation, microscopic studies were performed using an inverted microscope (Olympus, IX70, 20x magnification). This phenomenon was visualized using a solution seeded with RBC-sized magnetic-responsive micro-beads, having a diameter of 4µm or 8µm, in capillary tubes having a diameter of 50 µm or 100µm. FIG. 12 shows an example of flocculation formed in a 2,000 micro-beads/µl analog solution. Flocculation initially formed at the leading edge of the magnet (corresponding to the greatest magnetic field gradient.) The size of the flocculation grew over the entire length of the magnetic field region. When the size of the flocculation covered the length of the magnetic field region, the flocculation behaved as a fluidized bed. Magnetic-responsive micro-beads downstream were released, while upstream incoming magnetic-responsive micro-beads were added to the flocculation.

Example 2 - *pScreen*™ Prototype Fabrication and Testing

(A) Fabrication

Two sets of *pScreen*™ prototypes were fabricated: (1) a bench top prototype with multiple micro-channels for simultaneous testing of various samples; and (2) a single-use, portable, lab-on-card device. The *pScreen*™ bench-top prototype was described in the previous section. The *pScreen*™ lab-on-card prototype was realized using standard microfluidics fabrication techniques. In brief, the micro-channels were etched using a laser etcher system on a poly(ethylene terephthalate) glycol (Petg) substrate. The channels then were sealed using a matching poly(lactic-co-glycolic acid) (PLGA) top by thermal bonding using a hot press. The magnetic field gradient was obtained by placing two small magnets in an N-S configuration underneath the test channel. The *pScreen*™ lab-on-card prototype also was fabricated using injection cast molding in which the prototype was fabricated in poly(methyl methacrylate) (PMMA).

(B) Experimental Data for a Microfluidic Device for Detecting and Quantifying the Concentration of Magnetic Micro-beads

Two *pScreen*™ prototypes were tested using a variety of fluids such as, without limitation, blood, blood-analogs, or PBS buffer solution with different concentrations of surfactant. Tests were conducted using magnetic-responsive micro beads having a diameter of 4.1 µm or 8 µm. Sample concentrations between 100 micro-beads/µl and 200,000 micro-beads/µl were used. The concentration of magnetic-responsive micro-beads was determined by recording the level of the fluid on the calibration and test graduated column scales. Each mark on the scale corresponded to a given amount of fluid volume which flowed through the micro-channels. The relationship derived in Equation 8 was applied to convert the recorded volumes in magnetic-responsive micro-bead concentration. The analytic expression of the relationship between the volumes $Vo$ and $Vm$, specific for the tested prototypes, was derived by computing Equations (5) through (8), with $\hat{Q}$ given in Equations (9) through (11). To be especially noted is the fact that all of the equations provided above do not include time as a variable. Hence, it is not necessary to monitor/read the device's result at any specific time. The device reading at any time provides the same read-out. FIG. 13 is a graph showing magnetic-responsive micro-bead concentration measured using the *pScreen*™ device (y-axis) of the present invention versus magnetic-responsive micro-bead concentration measured with a standard hemocytometer (x-axis).

(C) Experimental Data for a *pScreen*™ Immunoassay

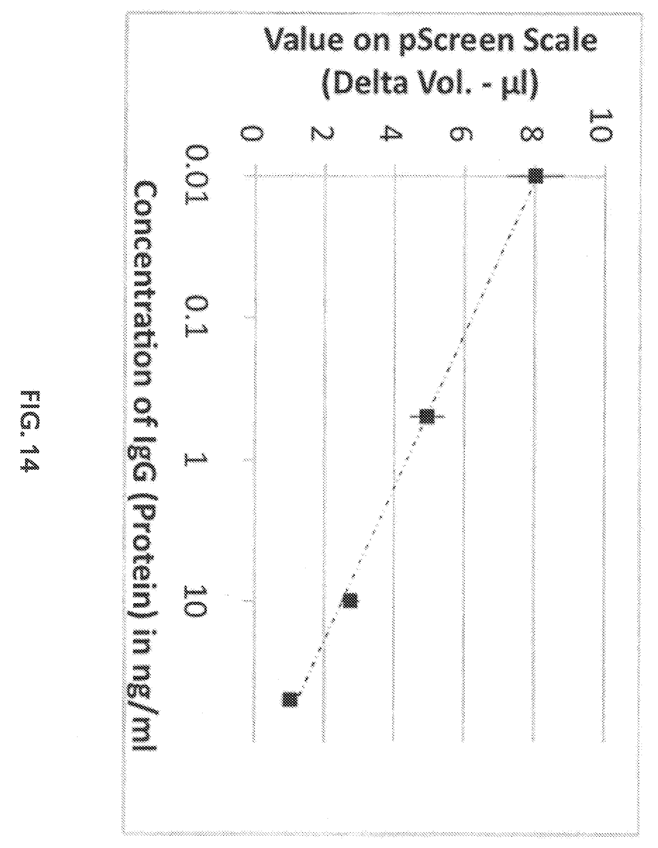

Several *pScreen*™ immunoassay prototypes were tested using buffer solutions containing various concentrations of mouse-IgG antibody prepared by titration from a known concentration IgG standard. The concentration of mouse-IgG antibody ranged from 0.5 ng/ml to 100 ng/ml. Tests were conducted using magnetic-responsive micro-beads coated with anti-mouse IgG antibody and coating the surface of a reaction chamber with anti-mouse IgG antibody. Sample volume ranged from 30 µl to 60 µl. The IgG antibody concentration was determined by recording the level of the fluid on the calibration and test graduated column scales. Each mark on the scale corresponded to a given amount of fluid volume which flowed through the micro-channels. FIG. 14 is a graph showing the difference between the volume collected in the control column ($Vo$) and the volume collected in the test column ($Vm$) (y-axis) versus the known concentration of IgG antibody (x-axis).

**Claims**

1. A microfluidic immunoassay device for detecting and measuring an analyte in a liquid sample, comprising a liquid sample inlet defined by an opening for accepting the liquid sample, said liquid sample inlet in continuous fluid connection with a flow resistor channel, said flow resistor channel in continuous fluid connection with an assay inlet of a reaction chamber, wherein the reaction chamber has adsorbed on its surface a plurality of immobilized antigen-specific antibodies (Ab1) specific to an analyte, wherein the surface of the reaction chamber also has a plurality of magnetic-responsive micro-beads desiccated thereon, each of said plurality of magnetic-responsive micro-beads coated with an antigen-specific antibody (Ab2) specific to the analyte, wherein the plurality of antibody-coated magnetic-responsive micro-beads rehydrate, disperse and bind to any analyte present in the liquid sample when the liquid sample flows through the reaction chamber, wherein any analyte present in the liquid sample also binds to the antigen-specific antibodies immobilized on the surface of the reaction chamber to form Ab1-analyte-Ab2-coated magnetic-responsive micro-bead complexes, wherein any unbound antibody-coated magnetic-responsive micro-beads exit the reaction chamber through an assay outlet, said assay outlet in continuous fluid connection with a micro-channel splitter which bifurcates to form a calibration micro-channel ($Co$) and at least one test micro-channel ($Cm$), said at least one test micro-channel and said calibration micro-channel kept at an equal and constant pressure, said calibration micro-channel in continuous fluid connection with a calibration graduated column, and said at least one test micro-channel in continuous fluid connection with at least one test graduated column, wherein one or more magnets creates a magnetic field gradient, said at least one test micro-channel exposed to the magnetic field gradient which causes flocculation of the magnetic-responsive micro-beads in the at least one test micro-channel which reduces flow rate ($Qm$) of the liquid sample in the at least one test micro-channel compared to flow rate ($Qo$) of the liquid sample in the calibration micro-channel, wherein each of the graduated columns has a graduated scale thereon which provides a read-out of the sample volume flowing through the test micro-channel, $Vm$, and the sample volume flowing through the calibration micro-channel, $Vo$, and collected in each of the graduated columns, which read-out is proportional to the concentration, $\rho$, of Ab1-analyte-Ab2-coated magnetic-responsive micro-bead complexes, which is a function of the concentration of the analyte in the liquid sample

2. The microfluidic immunoassay device of claim 1, wherein the ratio $Qm/Qo$, the difference $Qo-Qm$, and the ratio ($Qo-Qm$)$^p$/($Qm$)$^q$ are calculated, said ratios a proxy for the number of magnetic-responsive micro-beads in the liquid sample, which is a proxy for the concentration of analyte in the liquid sample.

3. The microfluidic immunoassay device of claim 1, wherein the magnetic field gradient is generated by two magnets aligned lengthwise with the at least one test micro-channel and along opposite poles to expose the at least one test micro-channel to the magnetic field gradient, said at least one test micro-channel located between a gap formed between the opposite poles of the magnets; or is generated by one magnet and a magnetic-responsive structure positioned near the at least one test micro-channel, wherein the magnetic field generated is between about 0.05 Tesla (T) to about 0.5 T, and wherein the magnetic field gradient generated is about 10 T/m or greater.

4. The microfluidic immunoassay device of claim 1, wherein the total sample volume collected in the calibration micro-channel graduated column serves as a control for parameters such as variation in viscosity between samples, level of hematocrit in blood samples, temperature and humidity fluctuations and sample volumes.

5. The microfluidic immunoassay device of claim 1, wherein each of the graduated columns has a length of about 20 mm to about 200 mm, a width of about 0.2 mm to about 3.0 mm, and a depth of about 0.1 mm to about 1.0 mm, wherein the respective graduated scales thereon each have a length and a cross-section that is visible to the naked eye, and wherein the read-out, providing a measurement of the unbound magnetic-responsive micro-beads and hence of the concentration of analyte present in the liquid sample, can be obtained at any time by direct comparison of the total sample volume visualized in the calibration graduated column with the at least one test graduated column.

6. The microfluidic immunoassay device of claim 1, wherein the liquid sample is selected from the group consisting of water, plasma, serum, buffer solution, urine, whole blood, blood analogs, liquid solutions from dilution of solid biological matter, and other biological fluids.

7. The microfluidic immunoassay device of claim 1, wherein the analyte is selected from the group consisting of proteins, protein fragments, antigens, antibodies, antibody fragments, peptides, RNA, RNA fragments, functionalized magnetic micro-beads specific to CD$^{4+}$, CD$^{8+}$cells, malaria-infected red blood cells, cancer cells, cancer biomarkers such as prostate specific antigen and other cancer biomarkers, viruses, bacteria such as *E. coli*, and other pathogenic agents.

8. The microfluidic immunoassay device of claim 1, wherein the device is a single use, portable, lab-on-card device fabricated by methods selected from the group consisting of etching each of the micro-channels on a poly(ethylene terephthalate) glycol plastic substrate, using a laser etcher system and then sealing the top of each of the micro-channels in a poly(lactic-co-glycolic acid) plastic by thermal bonding, and by injection mold casting in a poly(methyl methacrylate) plastic.

9. The microfluidic immunoassay device of claim 1, wherein the concentration of magnetic-responsive micro-beads which can be detected and quantified is about 50 micro-beads/$\mu$l to about 2 x $10^6$ micro-beads/$\mu$l, wherein the diameter of the magnetic-responsive micro-beads is about 0.2 $\mu$m to about 20 $\mu$m, wherein the at least one test micro-channel and the calibration micro-channel are made of a capillary tube having a length of about 0.2 cm to about 20 cm.

10. The microfluidic immunoassay device of claim 1, wherein bifurcation of the micro-channel splitter forms one test micro-channel and one calibration micro-channel, three test micro-channels and one calibration micro-channel, or four test micro-channels and one calibration micro-channel, wherein the one test micro-channel and the one calibration micro-channel have the same inner diameter of about 50 $\mu$m to about 500 $\mu$m, wherein each of the three test micro-channels is in continuous fluid connection with one graduated column, wherein the three test micro-channels each have a different inner diameter, wherein the one calibration micro-channel associated with the three micro-channels has an inner diameter such that the areas of the cross-section of the one calibration micro-channel is identical to the sum of the areas of the cross-sections of the three test micro-channels, wherein the three test micro-channels have an inner diameter of about 50 $\mu$m to about 500 $\mu$m, about 100 $\mu$m to about 250 $\mu$m, and about 250 $\mu$m to about 5 mm, respectively, wherein the four test micro-channels merge to be in continuous fluid connection with one graduated column, wherein the four test micro-channels have the same inner diameter of about 12.5 $\mu$m to about 125 $\mu$m, and wherein the one calibration micro-channel associated with the four test micro-channels has an inner diameter such that the area of the cross-section of the calibration micro-channel is identical to the sum of the areas of the cross-sections of the four test micro-channels.

11. A method for detecting and quantifying concentration of an analyte in a liquid sample, comprising:

adding a liquid sample to a liquid sample inlet of a reaction chamber, said liquid sample inlet defined by an opening for accepting the liquid sample, said reaction chamber having adsorbed on its surface a plurality of immobilized antigen-specific antibodies (Ab1) specific to an analyte, said surface of the reaction chamber also having a plurality of magnetic-responsive micro-beads desiccated thereon, each of said plurality of magnetic-responsive micro-beads coated with an antigen-specific antibody (Ab2) specific to the analyte;

having the liquid sample flow through the reaction chamber causing rehydration of the plurality of antibody-coated magnetic-responsive micro-beads as the liquid sample flows through the reaction chamber, said rehydration dispersing the antibody-coated magnetic-responsive micro-beads into the liquid sample;

binding the rehydrated antibody-coated magnetic-responsive micro-beads as well as the antigen-specific antibodies immobilized on the surface of the reaction chamber to any analyte present in the liquid sample to form Ab1-analyte-Ab2-coated magnetic micro-bead complexes on the surface of the reaction chamber;

having the liquid sample containing any unbound antibody-coated magnetic micro-beads exit the reaction chamber through an reaction chamber outlet, said assay outlet in continuous fluid connection with a micro-channel splitter which bifurcates to form a calibration micro-channel ($Co$) and at least one test micro-channel ($Cm$), said at least one test micro-channel and said calibration micro-channel kept at an equal and constant pressure, said calibration micro-channel in continuous fluid connection with a graduated column, and said at least one test micro-channel in continuous fluid connection with at least one graduated column, said each of the graduated columns having a graduated scale thereon; and

measuring flow rate ($Qm$) of the liquid sample in the at least one test micro-channel exposed to a magnetic field gradient with flow rate ($Qo$) of the fluid in the calibration micro-channel not exposed to a magnetic field gradient, wherein the presence of any unbound antibody-coated magnetic-responsive micro-beads in the at least one test micro-channel which is exposed to the magnetic field gradient causes flocculation of the antibody-coated magnetic-responsive micro-beads in the liquid sample which reduces the flow rate of the liquid sample through the at least one test micro-channel; and

calculating the ratio $Qm/Qo$, the difference $Qo-Qm$, and the ratio $(Qo-Qm)^p/(Qm)^q$, wherein $p$ and $q$ are derived through a calibration process, and wherein the ratios $Qm/Qo$ and $(Qo-Qm)^p/(Qm)^q$ are a proxy for the number of magnetic-responsive micro-beads in the liquid sample, which is a proxy for the concentration of analyte in the liquid sample.

12. The method of claim 11, wherein the magnetic field gradient is generated from two magnets aligned lengthwise with the at least one test micro-channel and along opposite poles to expose the at least one test micro-channel to the magnetic field gradient, said at least one test micro-channel located between a gap formed between the opposite poles of the magnets; or is generated from one magnet and a magnetic-responsive structure positioned near the at least one test micro-channel, wherein the magnetic field generated is between about 0.05 Tesla (T) to about 0.5 T, and wherein the magnetic field gradient generated is about 10 T/m or greater.

13. The method of claim 11, wherein the at least one test micro-channel and the calibration micro-channel are made of a capillary tube having a length of about 0.2 cm to about 20 cm, wherein the concentration of magnetic-responsive micro-beads which can be detected and quantified is about 50 micro-beads/$\mu$l to about 2 x $10^6$ micro-beads/$\mu$l, and wherein the diameter of the magnetic-responsive micro-beads is about 0.2 $\mu$m to about 20 $\mu$m.

14. The method of claim 11, wherein bifurcation of the micro-channel splitter forms one test micro-channel and one calibration micro-channel, three test micro-channels and one calibration micro-channel, or four test micro-channels and one calibration micro-channel, wherein the one test micro-channel and the one calibration micro-channel have the same inner diameter of about 50 $\mu$m to about 500 $\mu$m, wherein each of the three test micro-channels is in continuous fluid connection with one graduated column, wherein the three test micro-channels each have a different inner diameter, wherein the one calibration micro-channel associated with the three micro-channels has an inner diameter such that the areas of the cross-section of the one calibration micro-channel is identical to the sum of the areas of the cross-sections of the three test micro-channels, wherein the three test micro-channels have an inner diameter of about 50 $\mu$m to about 500 $\mu$m, about 100 $\mu$m to about 250 $\mu$m, and about 250 $\mu$m to about 5 mm, respectively, wherein the four test micro-channels merge to be in continuous fluid connection with one graduated column, wherein the four test micro-channels have the same inner diameter of about 12.5 $\mu$m to about 125 $\mu$m, and wherein the one calibration micro-channel associated with the four test micro-channels has an inner diameter such that the area of the cross-section of the calibration micro-channel is identical to the sum of the areas of the cross-sections of the four test micro-channels.

15. The method of claim 11, wherein the analyte is selected from the group consisting of proteins, protein fragments, antigens, antibodies, antibody fragments, peptides, RNA, RNA fragments, functionalized magnetic micro-beads specific to $CD^{4+}$, $CD^{8+}$ cells, malaria-infected red blood cells, cancer cells, cancer biomarkers such as prostate specific antigen and other cancer biomarkers, viruses, bacteria such as *E. coli*, and other pathogenic agents.

**Patentansprüche**

1. Mikrofluidische Immunoassay-Vorrichtung zum Nachweisen und Messen eines Analyten in einer Flüssigprobe mit einem Flüssigprobeneinlass, der durch eine Öffnung zur Aufnahme der Flüssigprobe definiert ist, wobei der Flüssigprobeneinlass in ununterbrochener Fluidverbindung mit einem Fließwiderstandskanal ist, wobei der Fließwiderstandskanal in ununterbrochener Fluidverbindung mit einem Assay-Einlass einer Reaktionskammer ist, wobei die Reaktionskammer an ihrer Oberfläche eine Vielzahl immobilisierter antigenspezifischer Antikörper (Ab1) adsorbiert hat, die für einen Analyten spezifisch sind, wobei die Oberfläche der Reaktionskammer außerdem eine Vielzahl von auf Magnetismus reagierenden Mikrokügelchen aufweist, die darauf getrocknet sind, wobei jedes der Vielzahl von auf Magnetismus reagierenden Mikrokügelchen mit einem antigenspezifischen Antikörper (Ab2) beschichtet ist, der für den Analyten spezifisch ist, wobei die Vielzahl von antikörperbeschichteten, auf Magnetismus reagierenden Mikrokügelchen rehydratisiert, dispergiert und an einen in der Flüssigprobe vorhandenen Analyten bindet, wenn die Flüssigprobe durch die Reaktionskammer fließt, wobei ein in der Flüssigprobe vorhandener Analyt auch an die an der Oberfläche der Reaktionskammer immobilisierten antigenspezifischen Antikörper bindet, um Ab1-Analyt-Ab2-beschichtete, auf Magnetismus reagierende Mikrokügelchen-Komplexe zu bilden, wobei ungebundene antikörperbeschichtete, auf Magnetismus reagierende Mikrokügelchen durch einen Assay-Auslass aus der Reaktionskammer austreten, wobei der Assay-Auslass in ununterbrochener Fluidverbindung mit einem Mikrokanalverzweiger ist, der sich gabelt, um einen Kalibrationsmikrokanal (*Co*) und mindestens einen Testmikrokanal (*Cm*) zu bilden, wobei mindestens ein Testmikrokanal und der Kalibrationsmikrokanal auf gleichem und konstantem Druck gehalten werden, wobei der Kalibrationsmikrokanal in ununterbrochener Fluidverbindung mit einer Kalibriersäule mit Skaleneinteilung ist und der mindestens eine Testmikrokanal in ununterbrochener Fluidverbindung mit mindestens einer Testsäule mit Skaleneinteilung ist, wobei ein oder mehrere Magneten einen Magnetfeldgradienten erzeugen, wobei mindestens ein Testmikrokanal dem Magnetfeldgradienten ausgesetzt ist, der eine Ausflockung der auf Magnetismus reagierenden Mikrokügelchen in dem mindestens einen Testmikrokanal bewirkt, die den Durchsatz *(Qm)* der Flüssigprobe in dem mindestens einen Testmikrokanal im Vergleich zum Durchsatz (*Qo*) der Flüssigprobe im Kalibrationsmikrokanal verringert, wobei auf jeder der Säulen mit Skaleneinteilung eine Maßskala ist, die einen Ablesewert des Probenvolumens, das durch den Testmikrokanal *Vm* fließt, und des Probenvolumens, das durch den Kalibrationsmikrokanal Vo fließt, und in jeder der Säulen mit Skaleneinteilung erfasst wurde, bereitstellt, wobei dieser Ablesewert proportional zur Konzentration ρ der Ab1-Analyt-Ab2-beschichteten, auf Magnetismus reagierenden Mikrokügelchen-Komplexe ist, die eine Funktion der Konzentration des Analyten in der Flüssigprobe ist.

2. Mikrofluidische Immunoassay-Vorrichtung nach Anspruch 1, wobei das Verhältnis *Qm/Qo*, die Differenz *Qm-Qo* und das Verhältnis $(Qo-Qm)^p/(Qm)^q$ berechnet werden, wobei die Verhältnisse eine Stellvertretung für die Zahl der auf Magnetismus reagierenden Mikrokügelchen in der Flüssigprobe sind, die eine Stellvertretung für die Konzentration des Analyten in der Flüssigprobe ist.

3. Mikrofluidische Immunoassay-Vorrichtung nach Anspruch 1, wobei der Magnetfeldgradient mittels zweier Magneten erzeugt wird, die mit entgegengesetzten Polen längs zu dem mindestens einen Testmikrokanal ausgerichtet sind, um den mindestens einen Testmikrokanal dem Magnetfeldgradienten auszusetzen, wobei der mindestens eine Testmikrokanal sich zwischen einer Lücke befindet, die zwischen den entgegengesetzten Polen der Magneten ausgebildet ist; oder mittels eines Magneten und einer auf Magnetismus reagierenden Struktur, die in der Nähe des mindestens einen Testmikrokanals angeordnet ist, erzeugt wird, wobei das Magnetfeld, das erzeugt wird, zwischen etwa 0,05 Tesla (T) und etwa 0,5 T liegt und wobei der Magnetfeldgradient, der erzeugt wird, etwa 10 T/m oder größer ist.

4. Mikrofluidische Immunoassay-Vorrichtung nach Anspruch 1, wobei das Gesamtvolumen der Probe, das sich in der Säule mit Skaleneinteilung des Kalibrationsmikrokanals ansammelt, als Kontrolle für Parameter wie etwa Viskositätsänderung von einer Probe zur anderen, Hämatokritwert in Blutproben, Temperatur- und Feuchtigkeitsschwankungen und Probenvolumen dient.

5. Mikrofluidische Immunoassay-Vorrichtung nach Anspruch 1, wobei jede der Säulen mit Skaleneinteilung eine Länge von etwa 20 mm bis etwa 200 mm, eine Breite von etwa 0,2 mm bis etwa 3,0 mm und eine Tiefe von etwa 0,1 mm bis etwa 1,0 mm aufweist, wobei die jeweiligen Maßskalen darauf jeweils eine Länge und einen Querschnitt aufweisen, die mit bloßem Auge sichtbar sind, und wobei der Ablesewert, der ein Messergebnis für die ungebundenen, auf Magnetismus reagierenden Mikrokügelchen und folglich die Konzentration des in der Flüssigkeitsprobe vorhandenen Analyten bereitstellt, jederzeit durch direkten Vergleich des Gesamtvolumens der Probe, sichtbar gemacht in der Kalibriersäule mit Skaleneinteilung, mit der mindestens einen Testsäule mit Skaleneinteilung erhalten werden kann.

6. Mikrofluidische Immunoassay-Vorrichtung nach Anspruch 1, wobei die Flüssigprobe aus der Gruppe ausgewählt ist, die aus Wasser, Plasma, Serum, Pufferlösung, Urin, Vollblut, Blutanaloga, flüssigen Lösungen infolge Verdünnung fester biologischer Materialien und anderen biologischen Flüssigkeiten besteht.

7. Mikrofluidische Immunoassay-Vorrichtung nach Anspruch 1, wobei der Analyt aus der Gruppe ausgewählt ist, die aus Proteinen, Proteinfragmenten, Antigenen, Antikörpern, Antikörperfragmenten, Peptiden, RNA, RNA-Fragmenten, funktionalisierten magnetischen Mikrokügelchen, die spezifisch für $CD^{4+}$, $CD^{8+}$-Zellen, mit Malaria infizierte rote Blutkörperchen, Krebszellen sind, Krebs-Biomarkern wie etwa prostataspezifisches Antigen oder anderen Krebs-Biomarkern, Viren, Bakterien wie etwa E. coli und anderen Krankheitserregern besteht.

8. Mikrofluidische Immunoassay-Vorrichtung nach Anspruch 1, wobei die Vorrichtung eine portable Westentaschenlabor- (Lab-on-Card-) Vorrichtung zum Einmalgebrauch ist, die durch Verfahren, die aus der Gruppe ausgewählt sind, die aus Ätzen jedes der Mikrokanäle auf einem mit Glykol modifizierten Poly(ethylenterephthalat)-Kunststoffsubstrat, Verwenden eines Lasergravursystems und dann Verschließen des oberen Endes jedes der Mikrokanäle mit einem Poly(milch-co-glykolsäure)-Kunststoff durch Heißverschweißen besteht, und durch Spritzgießen aus einem Poly(methylmethacrylat)-Kunststoff hergestellt ist.

9. Mikrofluidische Immunoassay-Vorrichtung nach Anspruch 1, wobei die Konzentration der auf Magnetismus reagierenden Mikrokügelchen, die nachgewiesen und quantitativ bestimmt werden kann, etwa 50 Mikrokügelchen/μl bis etwa $2 \times 10^6$ Mikrokügelchen/μl beträgt, wobei der Durchmesser der auf Magnetismus reagierenden Mikrokügelchen etwa 0,2 μm bis etwa 20 μm beträgt, wobei der mindestens eine Testmikrokanal und der Kalibrationsmikrokanal aus einem Kapillarröhrchen mit einer Länge von etwa 0,2 cm bis etwa 20 cm hergestellt sind.

10. Mikrofluidische Immunoassay-Vorrichtung nach Anspruch 1, wobei eine Gabelung des Mikrokanalverzweigers einen Testmikrokanal und einen Kalibrationsmikrokanal, drei Testmikrokanäle und einen Kalibrationsmikrokanal oder vier Testmikrokanäle und einen Kalibrationsmikrokanal bildet, wobei der eine Testmikrokanal und der eine Kalibrationsmikrokanal den gleichen Innendurchmesser von etwa 50 μm bis etwa 500 μm aufweisen, wobei jeder der drei Testmikrokanäle in ununterbrochener Fluidverbindung mit einer Säule mit Skaleneinteilung ist, wobei die drei Testmikrokanäle jeweils einen anderen Innendurchmesser aufweisen, wobei der eine Kalibrationsmikrokanal, der den drei Mikrokanälen zugeordnet ist, einen solchen Innendurchmesser aufweist, dass die Fläche des Querschnitts des einen Kalibrationsmikrokanals gleich der Summe der Flächen der Querschnitte der drei Testmikrokanäle ist, wobei die drei Testmikrokanäle einen Innendurchmesser von etwa 50 μm bis etwa 500 μm, etwa 100 μm bis etwa 250 μm bzw. etwa 250 μm bis etwa 5 mm aufweisen, wobei die vier Testmikrokanäle zusammenkommen, um in ununterbrochener Fluidverbindung mit einer Säule mit Skaleneinteilung zu sein, wobei die vier Testmikrokanäle den gleichen Innendurchmesser von etwa 12,5 μm bis etwa 125 μm aufweisen und wobei der eine Kalibrationsmikrokanal, der den vier Testmikrokanälen zugeordnet ist, einen solchen Innendurchmesser aufweist, dass die Fläche des Querschnitts des Kalibrationsmikrokanals gleich der Summe der Flächen der Querschnitte der vier Testmikrokanäle ist.

11. Verfahren zum Nachweisen und quantitativen Bestimmen der Konzentration eines Analyten in einer Flüssigprobe, umfassend:

Zugeben einer Flüssigprobe zu einem Flüssigprobeneinlass einer Reaktionskammer, wobei der Flüssigprobeneinlass durch eine Öffnung zum Aufnehmen der Flüssigprobe definiert ist, wobei die Reaktionskammer an ihrer Oberfläche eine Vielzahl immobilisierter antigenspezifischer Antikörper (Ab1) adsorbiert hat, die für einen Analyten spezifisch sind, wobei die Oberfläche der Reaktionskammer außerdem eine Vielzahl von auf Magnetismus reagierenden Mikrokügelchen aufweist, die darauf getrocknet sind, wobei jedes der Vielzahl von auf Magnetismus reagierenden Mikrokügelchen mit einem antigenspezifischen Antikörper (Ab2) beschichtet ist, der für den Analyten spezifisch ist;

Fließenlassen der Flüssigprobe durch die Reaktionskammer, wodurch eine Rehydratisation der Vielzahl von antikörperbeschichteten, auf Magnetismus reagierenden Mikrokügelchen bewirkt wird, während die Flüssigprobe durch die Reaktionskammer fließt, wobei durch die Rehydratisation die antikörperbeschichteten, auf Magnetismus reagierenden Mikrokügelchen in der Flüssigprobe dispergiert werden;

Binden sowohl der rehydratisierten antikörperbeschichteten, auf Magnetismus reagierenden Mikrokügelchen als auch der antigenspezifischen Antikörper, die an der Oberfläche der Reaktionskammer immobilisiert sind, an in der Flüssigprobe vorhandenen Analyten, um an der Oberfläche der Reaktionskammer Ab1-Analyt-Ab2-beschichtete, auf Magnetismus reagierende Mikrokügelchen-Komplexe zu bilden;

Austretenlassen der Flüssigprobe, die ungebundene antikörperbeschichtete, auf Magnetismus reagierende Mikrokügelchen enthält, aus der Reaktionskammer und zwar durch einen Reaktionskammerauslass, wobei der Assay-Auslass in ununterbrochener Fluidverbindung mit einem Mikrokanalverzweiger ist, der sich gabelt, um einen Kalibrationsmikrokanal ($Co$) und mindestens einen Testmikrokanal ($Cm$) zu bilden, wobei der mindestens eine Testmikrokanal und der Kalibrationsmikrokanal auf gleichem und konstantem Druck gehalten werden, wobei der Kalibrationsmikrokanal in ununterbrochener Fluidverbindung mit einer Säule mit Skaleneinteilung ist und der mindestens eine Testmikrokanal in ununterbrochener Fluidverbindung mit mindestens einer Säule mit Skaleneinteilung ist, wobei auf jeder der Säulen mit Skaleneinteilung eine Maßskala ist; und

Messen des Durchsatzes ($Qm$) der Flüssigprobe in dem mindestens einen Testmikrokanal, der einem Magnetfeldgradienten ausgesetzt ist, bei einem Durchsatz ($Qo$) der Flüssigkeit im Kalibrationsmikrokanal, der keinem Magnetfeldgradienten ausgesetzt ist, wobei das Vorhandensein von ungebundenen antikörperbeschichteten, auf Magnetismus reagierenden Mikrokügelchen in dem mindestens einen Testmikrokanal, der dem Magnetfeldgradienten ausgesetzt ist, eine Ausflockung der antikörperbeschichteten, auf Magnetismus reagierenden Mikrokügelchen in der Flüssigprobe bewirkt, die den Durchsatz der Flüssigprobe durch den mindestens einen Testmikrokanal verringert; und

Berechnen des Verhältnisses $Qm/Qo$, der Differenz $Qm-Qo$ und des Verhältnisses $(Qo-Qm)^p/(Qm)^q$, wobei p und q durch einen Kalibriervorgang erlangt werden, und wobei die Verhältnisse $Qm/Qo$ und $(Qo-Qm)^p/(Qm)^q$ eine Stellvertretung für die Zahl der auf Magnetismus reagierenden Mikrokügelchen in der Flüssigprobe sind, die eine Stellvertretung für die Konzentration des Analyten in der Flüssigprobe ist.

12. Verfahren nach Anspruch 11, wobei der Magnetfeldgradient von zwei Magneten erzeugt wird, die mit entgegengesetzten Polen längs zu dem mindestens einen Testmikrokanal ausgerichtet sind, um den mindestens einen Testmikrokanal dem Magnetfeldgradienten auszusetzen, wobei der mindestens eine Testmikrokanal sich zwischen einer Lücke befindet, die zwischen den entgegengesetzten Polen der Magneten ausgebildet ist; oder von einem Magneten und einer auf Magnetismus reagierenden Struktur, die in der Nähe des mindestens einen Testmikrokanals angeordnet ist, erzeugt wird, wobei das Magnetfeld, das erzeugt wird, zwischen etwa 0,05 Tesla (T) und etwa 0,5 T liegt und wobei der Magnetfeldgradient, der erzeugt wird, etwa 10 T/m oder größer ist.

13. Verfahren nach Anspruch 11, wobei der mindestens eine Testmikrokanal und der Kalibrationsmikrokanal aus einem Kapillarröhrchen mit einer Länge von etwa 0,2 cm bis etwa 20 cm hergestellt sind, wobei die Konzentration der auf Magnetismus reagierenden Mikrokügelchen, die nachgewiesen und quantitativ bestimmt werden kann, etwa 50 Mikrokügelchen/μl bis etwa $2 \times 10^6$ Mikrokügelchen/μl beträgt und wobei der Durchmesser der auf Magnetismus reagierenden Mikrokügelchen etwa 0,2 μm bis etwa 20 μm beträgt.

14. Verfahren nach Anspruch 11, wobei eine Gabelung des Mikrokanalverzweigers einen Testmikrokanal und einen Kalibrationsmikrokanal, drei Testmikrokanäle und einen Kalibrationsmikrokanal oder vier Testmikrokanäle und einen Kalibrationsmikrokanal bildet, wobei der eine Testmikrokanal und der eine Kalibrationsmikrokanal den gleichen Innendurchmesser von etwa 50 μm bis etwa 500 μm aufweisen, wobei jeder der drei Testmikrokanäle in ununterbrochener Fluidverbindung mit einer Säule mit Skaleneinteilung ist, wobei die drei Testmikrokanäle jeweils einen anderen Innendurchmesser aufweisen, wobei der eine Kalibrationsmikrokanal, der den drei Mikrokanälen zugeordnet ist, einen solchen Innendurchmesser aufweist, dass die Fläche des Querschnitts des einen Kalibrationsmikrokanals gleich der Summe der Flächen der Querschnitte der drei Testmikrokanäle ist, wobei die drei Testmikrokanäle einen Innendurchmesser von etwa 50 μm bis etwa 500 μm, etwa 100 μm bis etwa 250 μm bzw. etwa 250 μm bis etwa 5 mm aufweisen, wobei die vier Testmikrokanäle zusammenkommen, um in ununterbrochener Fluidverbindung mit einer Säule mit Skaleneinteilung zu sein, wobei die vier Testmikrokanäle den gleichen Innendurchmesser von etwa 12,5 μm bis etwa 125 μm aufweisen und wobei der eine Kalibrationsmikrokanal, der den vier Testmikrokanälen zugeordnet ist, einen solchen Innendurchmesser aufweist, dass die Fläche des Querschnitts des Kalibrationsmikrokanals gleich der Summe der Flächen der Querschnitte der vier Testmikrokanäle ist.

15. Verfahren nach Anspruch 11, wobei der Analyt aus der Gruppe ausgewählt ist, die aus Proteinen, Proteinfragmenten, Antigenen, Antikörpern, Antikörperfragmenten, Peptiden, RNA, RNA-Fragmenten, funktionalisierten magnetischen Mikrokügelchen, die spezifisch für $CD^{4+}$, $CD^{8+}$-Zellen, mit Malaria infizierte rote Blutkörperchen, Krebszellen sind, Krebs-Biomarkern wie etwa prostataspezifisches Antigen oder anderen Krebs-Biomarkern, Viren, Bakterien wie etwa E. coli und anderen Krankheitserregern besteht.

## Revendications

1. Dispositif d'immuno-essai microfluidique pour la détection et la mesure d'un analyte dans un échantillon liquide, comprenant une entrée d'échantillon liquide définie par une ouverture pour accepter l'échantillon liquide, ladite entrée d'échantillon liquide étant en connexion fluidique continue avec un canal de résistance à l'écoulement, ledit canal de résistance à l'écoulement étant en connexion fluidique continue avec une entrée d'essai d'une chambre de réaction, dans lequel la chambre de réaction a adsorbé sur sa surface une pluralité d'anticorps spécifiques à un antigène (Ab1) immobilisés spécifiques à un analyte, dans lequel la surface de la chambre de réaction a également une pluralité de microbilles sensibles au magnétisme desséchées sur celle-ci, chacune de ladite pluralité de microbilles sensibles au magnétisme étant revêtue par un anticorps spécifique à un antigène (Ab2) spécifique à l'analyte, dans lequel la pluralité de microbilles sensibles au magnétisme revêtues par un anticorps se réhydratent, se dispersent et se lient à tout analyte présent dans l'échantillon liquide lorsque l'échantillon liquide s'écoule à travers la chambre de réaction, dans lequel tout analyte présent dans l'échantillon liquide se lie également aux anticorps spécifiques à un antigène immobilisés sur la surface de la chambre de réaction pour former des complexes Ab1-analyte-microbilles sensibles au magnétisme revêtues par Ab2, dans lequel toutes les microbilles sensibles au magnétisme revêtues par un anticorps non liées quittant la chambre de réaction à travers une sortie d'essai, ladite sortie d'essai étant en connexion fluidique continue avec un diviseur de micro-canal qui bifurque pour former un micro-canal de calibrage (Co) et au moins un micro-canal de test (Cm), ledit au moins un micro-canal de test et ledit micro-canal de calibrage étant maintenus à une pression égale et constante, ledit micro-canal de calibrage étant en connexion fluidique continue avec une colonne graduée de calibrage, et ledit au moins un micro-canal de test étant en connexion fluidique continue avec au moins une colonne graduée de test, dans lequel un ou des aimants créent un gradient de champ magnétique, ledit au moins un micro-canal de test étant exposé au gradient de champ magnétique qui provoque une floculation des microbilles sensibles au magnétisme dans le au moins un micro-canal de test qui réduit le débit (Qm) de l'échantillon liquide dans le au moins un micro-canal de test par comparaison avec le débit (Qo) de l'échantillon liquide dans le micro-canal de calibrage, dans lequel chacune des colonnes graduées a une échelle graduée sur celle-ci qui fournit une lecture du volume d'échantillon s'écoulant à travers le micro-canal de test, Vm, et du volume d'échantillon s'écoulant à travers le micro-canal de calibrage, Vo, et collecté dans chacune des colonnes graduées, laquelle lecture est proportionnelle à la concentration, ρ, de complexes Ab1-analyte-microbilles sensibles au magnétisme revêtues par Ab2, qui est une fonction de la concentration de l'analyte dans l'échantillon liquide.

2. Dispositif d'immuno-essai microfluidique selon la revendication 1, dans lequel le rapport Qm/Qo, la différence Qo-Qm, et le rapport $(Qo-Qm)^p/(Qm)^q$ sont calculés, lesdits rapports étant un indicateur pour le nombre de microbilles sensibles au magnétisme dans l'échantillon liquide, qui est un indicateur pour la concentration d'analyte dans l'échantillon liquide.

3. Dispositif d'immuno-essai microfluidique selon la revendication 1, dans lequel le gradient de champ magnétique est généré par deux aimants alignés selon la longueur avec le au moins un micro-canal de test et le long de pôles opposés pour exposer le au moins un micro-canal de test au gradient de champ magnétique, ledit au moins un micro-canal de test étant situé entre un intervalle formé entre les pôles opposés des aimants ; ou est généré par un aimant et une structure sensible au magnétisme positionnée à proximité du au moins un micro-canal de test, le champ magnétique généré étant entre environ 0,05 Tesla (T) et environ 0,5 T, et le gradient de champ magnétique généré étant d'environ 10 T/m ou plus.

4. Dispositif d'immuno-essai microfluidique selon la revendication 1, dans lequel le volume d'échantillon total collecté dans la colonne graduée de micro-canal de calibrage sert de témoin pour des paramètres tels que la variation de viscosité entre les échantillons, le taux d'hématocrite dans les échantillons sanguins, les fluctuations de température et d'humidité et les volumes d'échantillon.

5. Dispositif d'immuno-essai microfluidique selon la revendication 1, dans lequel chacune des colonnes graduées a une longueur d'environ 20 mm à environ 200 mm, une largeur d'environ 0,2 mm à environ 3,0 mm, et une profondeur d'environ 0,1 mm à environ 1,0 mm, dans lequel les échelles graduées respectives sur celles-ci ont chacune une longueur et une section transversale qui est visible à l'oeil nu, et dans lequel la lecture, fournissant une mesure des microbilles sensibles au magnétisme non liées et de ce fait la concentration de l'analyte présent dans l'échantillon liquide, peut être obtenue à tout moment par comparaison directe du volume total d'échantillon visualisé dans la colonne graduée de calibrage avec la au moins une colonne graduée de test.

6. Dispositif d'immuno-essai microfluidique selon la revendication 1, dans lequel l'échantillon liquide est choisi dans le groupe consistant en eau, plasma, sérum, solution tampon, urine, sang total, analogues sanguins, solutions liquides provenant de la dilution d'une matière biologique solide, et autres liquides biologiques.

7. Dispositif d'immuno-essai microfluidique selon la revendication 1, dans lequel l'analyte est choisi dans le groupe consistant en protéines, fragments de protéines, antigènes, anticorps, fragments d'anticorps, peptides, ARN, fragments d'ARN, microbilles magnétiques fonctionnalisées spécifiques des cellules $CD^{4+}$, $CD^{8+}$, globules rouges infectés par la malaria, cellules cancéreuses, bio-marqueurs de cancer tels que l'antigène spécifique de la prostate et autres bio-marqueurs du cancer, virus, bactéries telles qu'*E. coli*, et autres agents pathogènes.

8. Dispositif d'immuno-essai microfluidique selon la revendication 1, dans lequel le dispositif est un dispositif à usage unique, portable, laboratoire sur carte, fabriqué par des méthodes choisies dans le groupe consistant en gravure de chacune des microcanaux sur un substrat de matière plastique poly(éthylène téréphtalate) glycol, à l'aide d'un système de gravure laser, puis scellement de la partie supérieure de chacun des micro-canaux dans une matière plastique acide poly(lactique-co-glycolique) par liaison thermique, et par coulée en moule d'injection dans une matière plastique poly(méthacrylate de méthyle).

9. Dispositif d'immuno-essai microfluidique selon la revendication 1, dans lequel la concentration de microbilles sensibles au magnétisme qui peut être détectée et quantifiée est d'environ 50 microbilles/$\mu$l est d'environ 2 x 10⁶ microbilles/$\mu$l, dans lequel le diamètre des microbilles sensibles au magnétisme est d'environ 0,2 $\mu$m à environ 20 $\mu$m, dans lequel le au moins un micro-canal de test et le micro-canal de calibrage sont faits d'un tube capillaire ayant une longueur d'environ 0,2 cm à environ 20 cm.

10. Dispositif d'immuno-essai microfluidique selon la revendication 1, dans lequel une bifurcation du diviseur de micro-canal forme un micro-canal de test et un micro-canal de calibrage, trois micro-canaux de test et un micro-canal de calibrage, ou quatre micro-canaux de test et un micro-canal de calibrage, dans lequel le un micro-canal de test et le un micro-canal de calibrage ont le même diamètre interne d'environ 50 $\mu$m à environ 500 $\mu$m, dans lequel chacun des trois micro-canaux de test est en connexion fluidique continue avec une colonne graduée, dans lequel les trois micro-canaux de test ont chacun un diamètre interne différent, dans lequel le micro-canal de calibrage associé aux trois micro-canaux a un diamètre interne tel que les aires de la section transversale du un micro-canal de calibrage sont identiques à la somme des aires des sections transversales des trois micro-canaux de test, dans lequel les trois micro-canaux de test ont un diamètre interne respectivement d'environ 50 $\mu$m à environ 500 $\mu$m, d'environ 100 $\mu$m à environ 250 $\mu$m et d'environ 250 $\mu$m à environ 5 mm, dans lequel les quatre micro-canaux de test fusionnant pour être en connexion fluidique continue avec une colonne graduée, dans lequel les quatre micro-canaux de test ont le même diamètre interne d'environ 12,5 $\mu$m à environ 125 $\mu$m, et dans lequel le un micro-canal de calibrage associé avec les quatre micro-canaux de test a un diamètre interne tel que l'aire de la section transversale du micro-canal de calibrage est identique à la somme des aires des sections transversales des quatre micro-canaux de test.

11. Procédé pour la détection et la quantification de la concentration d'un analyte dans un échantillon liquide, comprenant :

ajouter un échantillon liquide à une entrée d'échantillon liquide d'une chambre de réaction, ladite entrée d'échantillon liquide étant définie par une ouverture destinée à accepter l'échantillon liquide, ladite chambre de réaction ayant adsorbé sur sa surface une pluralité d'anticorps spécifiques à un antigène (Ab1) immobilisés, spécifiques à un analyte, ladite surface de la chambre de réaction ayant également une pluralité de microbilles sensibles au magnétisme desséchées sur celle-ci, chacune de ladite pluralité de microbilles sensibles au magnétisme étant revêtue par un anticorps spécifique à un antigène (Ab2) spécifique à l'analyte ;

amener l'échantillon liquide à s'écouler à travers la chambre de réaction provoquant une réhydratation de la pluralité de microbilles sensibles au magnétisme revêtues par un anticorps alors que l'échantillon liquide s'écoule à travers la chambre de réaction, ladite réhydratation dispersant les microbilles sensibles au magnétisme revêtues par un anticorps dans l'échantillon liquide ;

lier les microbilles sensibles au magnétisme revêtues par un anticorps réhydratées ainsi que les anticorps spécifiques d'un antigène immobilisés sur la surface de la chambre de réaction à tout analyte présent dans l'échantillon liquide pour former des complexes Ab1-analyte-microbilles magnétiques revêtues par Ab2 sur la surface de la chambre de réaction ;

amener l'échantillon liquide contenant toutes microbilles magnétiques revêtues par un anticorps non liées à quitter la chambre de réaction à travers une sortie de chambre de réaction, ladite sortie d'essai en connexion fluidique continue avec un diviseur de micro-canal qui bifurque pour former un micro-canal de calibrage ($Co$) et au moins un micro-canal de test ($Cm$), ledit au moins un micro-canal de test et ledit micro-canal de calibrage étant maintenus à une pression égale et constante, ledit micro-canal de calibrage étant en connexion fluidique continue avec une colonne graduée, et ledit au moins un micro-canal de test étant en connexion fluidique continue avec au moins une colonne graduée, ladite chacune des colonnes graduées ayant une échelle graduée sur celle-ci ; et

mesurer le débit ($Qm$) de l'échantillon liquide dans le au moins un micro-canal de test exposé à un gradient de champ magnétique avec un débit ($Qo$) du fluide dans le micro-canal de calibrage non exposé à un gradient de champ magnétique, la présence de toutes microbilles sensibles au magnétisme revêtues par un anticorps non liées dans le au moins un micro-canal de test qui est exposé au gradient de champ magnétique provoquant une floculation des microbilles sensibles au magnétisme revêtues par un anticorps dans l'échantillon liquide qui réduit le débit de l'échantillon liquide à travers le au moins un micro-canal de test ; et

calculer le rapport $Qm/Qo$, de la différence $Qo-Qm$, et le rapport $(Qo-Qm)^p/(Qm)^q$, où $p$ et $q$ sont déduits par un procédé de calibrage, et les rapports $Qm/Qo$ et $(Qo-Qm)^p/(Qm)^q$ étant un indicateur pour le nombre de microbilles sensibles au magnétisme dans l'échantillon liquide, qui est un indicateur pour la concentration de l'analyte dans l'échantillon liquide.

12. Procédé selon la revendication 11, dans lequel le gradient de champ magnétique est généré à partir de deux aimants alignés selon la longueur avec le au moins un micro-canal de test et le long de pôles opposés pour exposer le au moins un micro-canal de test au gradient de champ magnétique, ledit au moins un micro-canal de test étant situé entre un intervalle formé entre les pôles opposés des aimants ; ou est généré à partir d'un aimant et d'une structure sensible au magnétisme positionnée à proximité du au moins un micro-canal de test, le champ magnétique généré étant entre environ 0,05 Tesla (T) à environ 0,5 T, et le gradient de champ magnétique généré étant d'environ 10 T/m ou plus.

13. Procédé selon la revendication 11, dans lequel le au moins un micro-canal de test et le micro-canal de calibrage sont faits d'un tube capillaire ayant une longueur d'environ 0,2 cm à environ 20 cm, dans lequel la concentration de microbilles sensibles au magnétisme qui peut être détectée et quantifiée est d'environ 50 microbilles/$\mu$l à environ 2 x 10$^6$ microbilles/$\mu$l, et dans lequel le diamètre des microbilles sensibles au magnétisme est d'environ 0,2 $\mu$m à environ 20 $\mu$m.

14. Procédé selon la revendication 11, dans lequel une bifurcation du diviseur de micro-canal forme un micro-canal de test et un micro-canal de calibrage, trois micro-canaux de test et un micro-canal de calibrage, ou quatre micro-canaux de test et un micro-canal de calibrage, dans lequel le un micro-canal de test et le un micro-canal de calibrage ont le même diamètre interne d'environ 50 $\mu$m à environ 500 $\mu$m, dans lequel chacun des trois micro-canaux de test est en connexion fluidique continue avec une colonne graduée, dans lequel les trois micro-canaux de test ont chacun un diamètre interne différent, dans lequel le un micro-canal de calibrage associé avec les trois micro-canaux a un diamètre interne tel que l'aire de la section transversale du un micro-canal de calibrage est identique à la somme des aires des sections transversales des trois micro-canaux de test, dans lequel les trois micro-canaux de test ont un diamètre interne respectivement environ de 50 μm à environ 500 μm, d'environ 100 μm à environ 250 μm, et d'environ 250 μm à environ 5 mm, dans lequel les quatre micro-canaux de test fusionnent pour être en connexion fluidique continue avec une colonne graduée, dans lequel les quatre micro-canaux de test ont le même diamètre interne d'environ 12,5 μm à environ 125 μm, et dans lequel le un micro-canal de calibrage associé avec les quatre micro-canaux de test a un diamètre interne tel que l'aire de la section transversale du micro-canal de calibrage est identique à la somme des aires des sections transversales des quatre micro-canaux de test.

15. Procédé selon la revendication 11, dans lequel l'analyte est choisi dans le groupe consistant en protéines, fragments de protéines, antigènes, anticorps, fragments d'anticorps, peptides, ARN, fragments d'ARN, microbilles magnétiques fonctionnalisées spécifiques des cellules $CD^{4+}$, $CD^{8+}$, globules rouges infectés par la malaria, cellules cancéreuses, bio-marqueurs de cancer tels qu'un antigène spécifique de la prostate et autres bio-marqueurs du cancer, virus, bactéries telles qu'*E. coli*, et autres agents pathogènes.

FIG. 1

FIG.2

FIG. 3

FIG. 4

EP 2 751 556 B1

FIG. 5

FIG. 6

EP 2 751 556 B1

FIG.7

29

FIG. 8

FIG. 9

EP 2 751 556 B1

**FIG. 10**

FIG. 11

FIG. 12

FIG. 13

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **DITTMER et al.** *Philips Research Europe*